# EUROPEAN PATENT APPLICATION

(11) **EP 4 509 603 A1**
(43) Date of publication of application: **19.02.2025**
(21) Application number: 23382843.3
(22) Date of filing: 12.08.2023
(51) Int. Cl.: C12N 15/115

(54) **NEW APTAMERS AGAINST EPHA2 (EPHRIN TYPE-A RECEPTOR 2)**

(71) Applicant: Aptadel Therapeutics SL., 08028 Barcelona (ES)
(72) Inventor: LORENTE-MARINA, Gisela, 08028 Barcelona (ES); FERNANDEZ-MIRANDA, Gonzalo, 08003 Barcelona (ES)
(74) Representative: Hoffmann Eitle

(57) **Abstract**

The present invention relates to new aptamers against EphA2 (ephrin type-A receptor 2), chemically modified versions thereof and/or complexes comprising any thereof, as well as compositions comprising any of these. It further pertains to their use in a method ofEphA2 detection or quantification, or alternatively in a method comprising EphA2 detection or quantification, such as for the screening, diagnosis, prognosis, or monitoring of a EphA2-related disease. It further pertains to the use of the nucleic acid aptamer, complex or composition in a prophylactic and/or therapeutic method for the treatment of a EphA2-related disease, such as EphA2 expressing cancers, including Ewing sarcoma, and to related kits.

## Description

### FIELD OF THE INVENTION

The present invention relates to the field of biomedicine. Specifically, it refers to new aptamers against EphA2 (ephrin type-A receptor 2), chemically modified versions thereof and/or complexes comprising any thereof, as well as compositions comprising any of these. It further pertains to their use in a method ofEphA2 detection or quantification, or alternatively in a method comprising EphA2 detection or quantification, such as for the screening, diagnosis, prognosis, or monitoring of a EphA2- related disease. It further pertains to the use of the nucleic acid aptamer, complex or composition in a prophylactic and/or therapeutic method for the treatment of a EphA2-related disease, such as EphA2 expressing cancers, including Ewing sarcoma, and to related kits.

### BACKGROUND OF THE INVENTION

Ephrin (Eph) receptors are the most extensive subfamily of receptor tyrosine-kinases involved in several processes, including angiogenesis, tissue-border formation, cell migration, and cell plasticity. These receptors are well-established mediators in cell-cell interactions and motility and are expressed in human cancers. Among these receptors, EphA2 (ephrin type-A receptor 2) has been implicated in many processes crucial to malignant progression, such as migration, invasion, metastasis, proliferation, survival, and angiogenesis. Inhibition of EphA2 has been reported to decrease tumor growth, survival, and tumor-induced angiogenesis in multiple preclinical models of breast, ovarian, and pancreatic cancers (Tandon et al. 2011, Kasinski and Slack 2013; and Quinn et al. 2016).

Various solid tumors have been reported to express high levels of EphA2, such as ovarian (Thaker, P.H., et al. 2004, Lin, Y.G., et al. 2007), prostate (Walker-Daniels, J., et al. 1999, Zhao, Y. et al. 2021), pancreatic (Duxbury, M.S., et al. 2004), glioblastoma (Wykosky, J., et al. 2005, Wang, L.F.,et al 2008), lung (Kinch, M.S., et al. 2003), melanoma (Margaryan, N.v., et 2009, Udayakumar, D.,et al 2011), esophageal (Miyazaki, T.,et al 2003), colorectal (Dunne, P.D., et al. 2016, Kataoka, H., et al. 2004, Saito, T., et al. 2004), bone sarcomas (Giordano, G., et al. 2021, Posthumadeboer, J., et al. 2013) and breast tumors (Zhang, X. 2021, Brantley-Sieders, D.M., et al. 2008, Zelinski, D.P., et al. 2001). Furthermore, overexpression of the EphA2 receptor has been associated to the aggressiveness and metastatic potential for many of these tumors (Zhao, Y., et al. 2021, Zelinski, D.P., et al. 2001, Brantley-Sieders, D.M., et al. 2005).

Ewing's sarcoma (ES) is a rare type of cancer that occurs in the bones or in the soft tissue around the bones. It accounts for about 35% of all sarcoma cases, being a major contributor to morbidity and mortality among cancers, especially in children. Approximately 25% of these patients have metastatic disease at the time of diagnosis and due to the lack of effective treatments, their survival at that time is only 20%. Conventional chemotherapies, such as vincristine, cyclophosphamide, and doxorubicin, currently represent the only drugs approved for the treatment of ES. These non-specific drugs confer limited efficacy and detrimental side effects due to their toxicity. In this context, it is necessary to develop new targeted, selective and effective solutions for the treatment of this devastating cancer.

Various strategies have been described to target and modulate EphA2 activation. Monoclonal antibodies have been developed that mimic the actions of ephrin A1 (Carles-Kinch, K.,et al. 2002, Yang, Y., et al. 2021) and target the large extracellular domain of EphA2 that is frequently upregulated by tumor cells (Biao-xue, R., et al. 2011). Peptides and antibodies that bind the ephrin-binding pocket have been used to specifically deliver cytotoxins, but their application is limited due to modest binding affinity (Jackson, D., et al. 2008, Koolpe, M., et al. 2002, Wang, S., et al. 2012).

Aptamers are short single-stranded nucleic acid oligomers (ssDNA or RNA) that form complex three-dimensional shapes and bind with high affinity and specificity to target molecules. Aptamers possess key advantages over antibodies, for instance, aptamers can be chemically synthesized and can be chemically modified to fine-tune their properties for specific applications such as increasing serum stability, improving pharmacokinetics, and delivering small molecules or imaging agents (Odeh, F., et al. 2019). In addition, chemically modified RNA aptamers may have little-to-no immunogenicity, resulting in increased safety for clinical application. Aptamers thus have become promising candidates for diagnostic and targeted therapeutic applications, typically in the form of aptamer-drug(s) conjugates.

WO2020245076 A1 describes a 2'-fluoro-modified pyrimidine RNA aptamer which specifically binds EphA2 for treating or diagnosing tumors that express EphA2. In particular, it describes that the RNA-aptamer of SEQ ID NO:1 was able to bind the EphA2 receptor and be internalized, providing an anti-cancer effect on its own. A more recent article also refers to an undisclosed 2'-fluoro-modified pyrimidine anti- EphA2 RNA aptamer and its antitumor properties, in particular in Ewing sarcoma (Santana-Viera et al. Molecular Therapy: Nucleic Acids Vol. 32 June 2023).

There is presently a need of finding new anti-EphA2 targeting molecules, such as aptamers, with low immunogenicity, improved stability, binding (e.g., affinity, avidity, specificity and/or selectivity), targeted cell internalization, tissue penetration, anti proliferative and/ or antimetastatic properties.

Besides, obtaining anti-EphA2 aptamers of 50 or less nucleotides is also desired since a shorter aptamer size is associated with lower synthesis errors and costs, as well as improved stability (Adachi T, et al. 2019).

### SUMMARY OF THE INVENTION

As described herein, the inventors have generated new aptamers which have been shown to specifically recognize human EphA2 tumor receptor with high binding and/or cell internalisation properties.

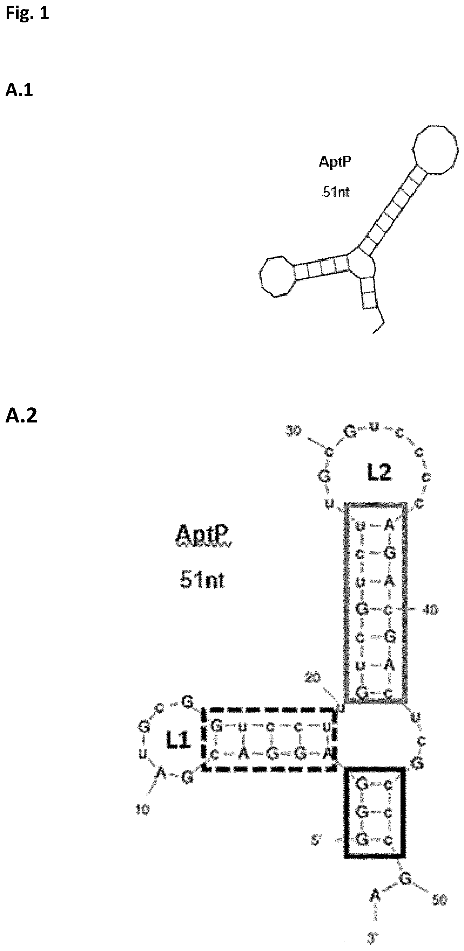

The 51 nt anti-EphA2 aptamer of sequence SEQ ID NO:2 (GGGAGGACGAUGCGGUCCU**U**GUCGUCUU**G**CGUCCCCAGACGACUCGCCCGA) described in WO2020245076 A1 (WO'076), is referred herein as AptP or SEQ ID NO: 109 and has been used for comparison purposes. The predicted conformational structure of AptP is illustrated in Fig.1A.

Indeed, the inventors have identified a series of anti-EphA2 aptamers (see Tables 2.1. and 2.2) characterized by comprising 5' to 3' the three regions identified in the first aspect of the invention as Region 1 (R1), Region 2 (R2), and Region 3 (R3) which were found to have improved binding and/or cell internalization properties with respect to the anti-EphA2 aptamer of SEQ ID NO:2 described in WO'076. In preferred embodiments, said aptamers are of formula (I) as defined herein.

This was completely unexpected since these aptamers have all in common a mutation with respect to AptP in the core region defined in WO'076 as SEQ ID NO: 1 (underlined above within SEQ ID NO:2 and referred herein as SEQ ID NO: 110), which was described by the authors of WO'076 to be a functional loop essential for the EphA2 binding activity, emphasizing that any substitutions had to be outside this region (see page 16, line 20 to page 17, line 8).

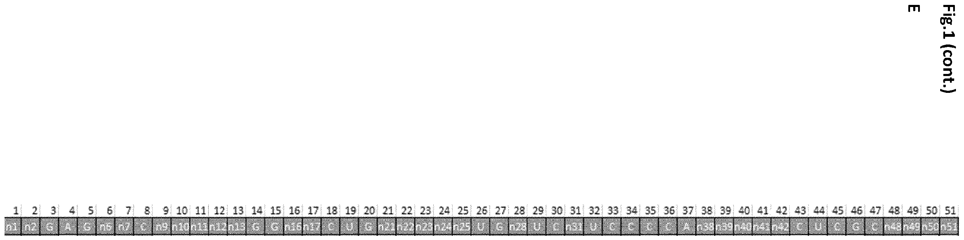

Indeed, the aptamers of the first aspect of the invention have two differing mutations in its Region 2 (R2) with respect to AptP, more specifically, the G before n21 and the U after n28. For instance, the aptamers of formula I (see Fig. 1E) differ from AptP at least in the presence of a "G" instead of a "U" at position 20 and a "U" instead of a G at position 29 of the aptamer of formula (I). These are highlighted in bold in SEQ ID NO: 2 of WO'076 above. It can be observed that the mutation at position 29 falls within the defined core region of AptP and position 20 is adjacent to it.

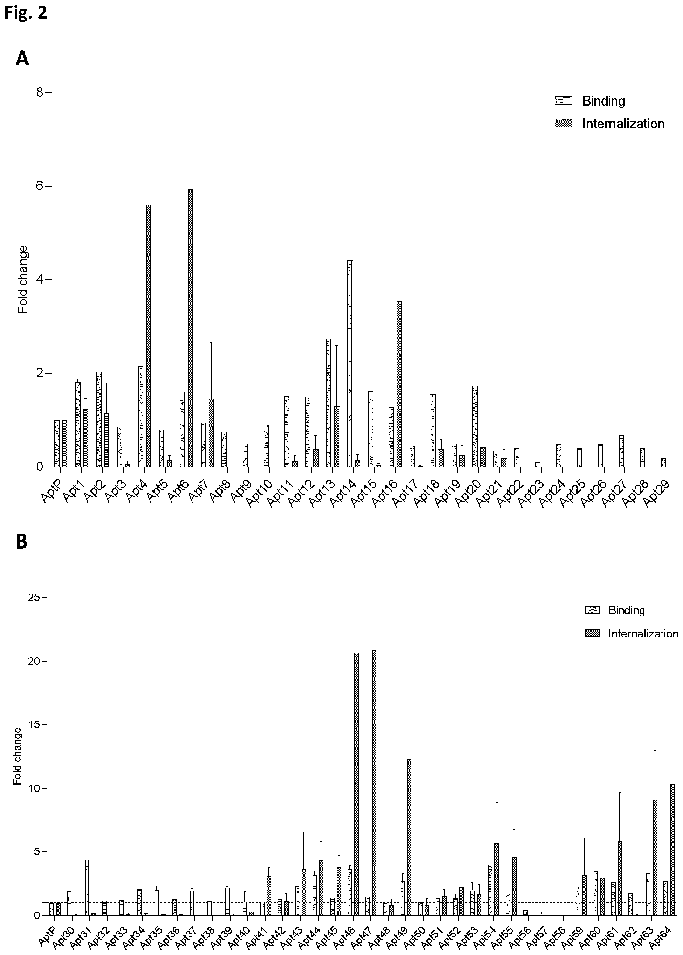

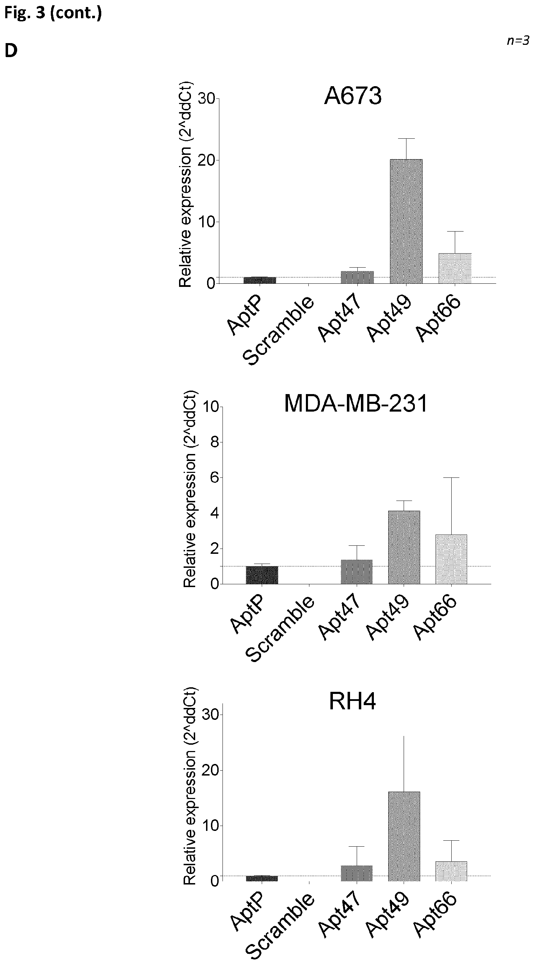

From the selected anti-EphA2 aptamers, the inventors have identified a subgroup of aptamers having exceptional cell internalization properties in EphA2 receptor expressing cell lines. Indeed, Apt4, Apt6, Apt46, Apt47, Apt49, Apt54, Apt61, Apt63, Apt64, Apt66 and Apt67 were shown an improvement of at least about 5-fold over AptP in Ewing sarcoma cell line A673 (Fig.2A-2C). From these, Apt46, Apt47, Apt49, and Apt64 were found to have even higher cell internalization properties reaching values of 10-fold or higher than AptP (Fig.2B). Cell internalization assays were also conducted for Apt47, Apt49 and Apt66 in other EphA2 receptor expressing cell lines, such as breast cancer and rhabdomysarcoma cell lines, Apt49 and Apt66 were shown to have cell internalization properties superior to AptP also in these cell lines (Fig.3D).

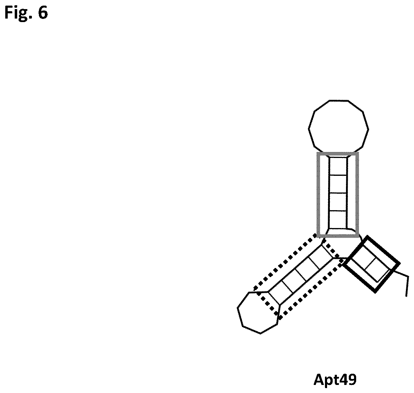

With regard to its binding properties (Fig.2A-2C), the following aptamers were found to have an improvement of at least about 2-fold with respect to Apt: Apt2, Apt4, Apt13, Apt14, Apt31, Apt34, Apt35, Apt39, Apt43, Apt44, Apt46, Apt49, Apt53, Apt54, Apt59, Apt60, Apt61, Apt63, Apt64, Apt65, Apt66, Apt67, Apt75, Apt78, Apt79, Apt86, Apt87. The most promising candidates were Apt14, Apt31, Apt54, Apt67 having an improvement of 4-fold or higher over AptP. The affinity properties of the Apt49, Apt54 and Apt60 candidates are shown in Figs. 6A-6C.

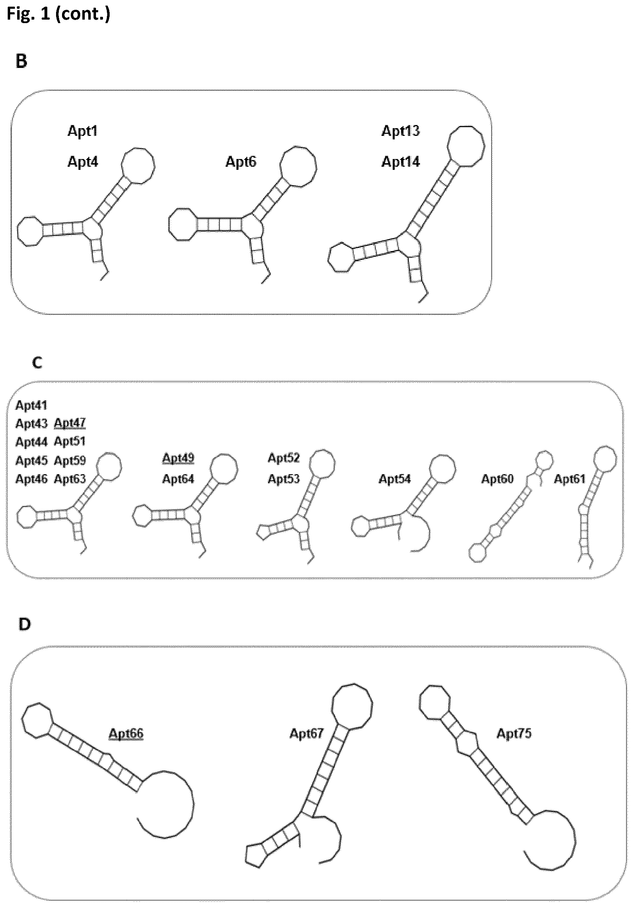

AptP is characterized by having a 3-stem structure as the predicted more stable folding. A representation of AptP three-dimensional structure is provided at Fig. 1.A.2. It was unexpectedly found that it was possible to almost completely delete the predicted loop 1 in AptP without loss of binding and/or cell internalization activity (Fig.2B-2C), for instance when two or more of n₉ to n₁₃ of the aptamer of formula (I) are absent, such as in Apt47, Apt51, Apt52, Apt53 and Apt66.

Aptamers with improved binding properties are of interest for detection (e.g., screening, diagnosis, prognosis or monitoring) and therapeutic or prophylactic treatment purposes. In addition, aptamers with good cell internalization properties are desired when the aptamer is conjugated to a functional moiety (e.g., an antisense oligonucleotide, siRNA, microRNA, shRNA or ribozyme) which will exert its activity intracellularly.

These new aptamers will enable the efficient and selective delivery of antitumor drugs to tumor cells without affecting healthy cells. Thus, specifically releasing the antitumor drug in the target tumor cell results in a decrease of undesired adverse effects with respect to non-targeted therapies.

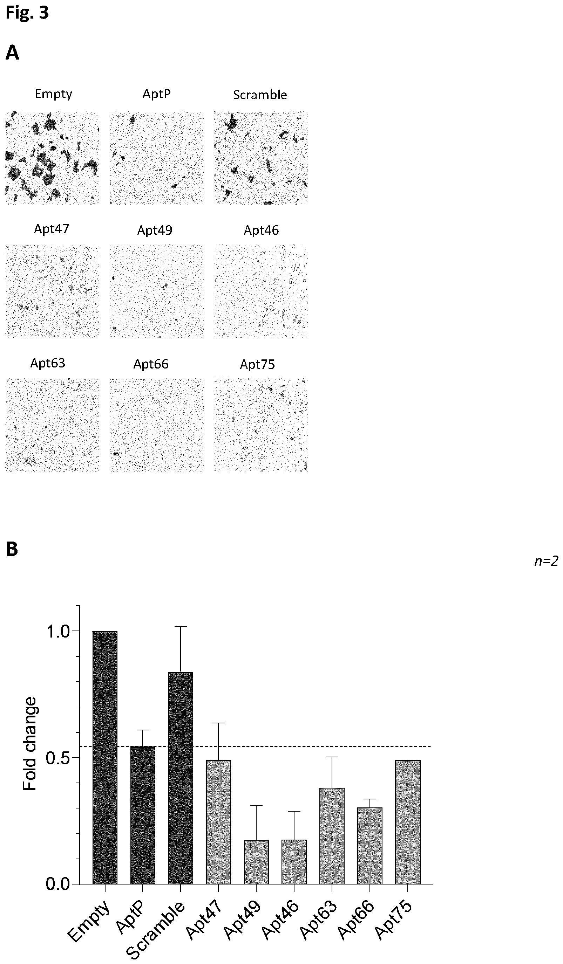

As discussed above, EphA2 has been previously described to have a role in cancer proliferation and metastasis. Some of the most promising candidates with respect to their binding and cell internalization properties, namely Apt46, Apt47, Apt49, Apt63, Apt66 and Apt75 were tested for their ability to inhibit migration of EphA2 expressing cancer cells (i.e., Ewing's sarcoma A673 cells). A673 cells treatment with all the tested aptamer candidates resulted in a significant reduction of the migratory and invasive capacity of A673 cells *in vitro*, compared to the untreated cells (Empty) (Fig.3A). This evidences the antimetastatic properties of the anti-EphA2 aptamers per se (i.e., without being conjugated to a functional moiety as described above).

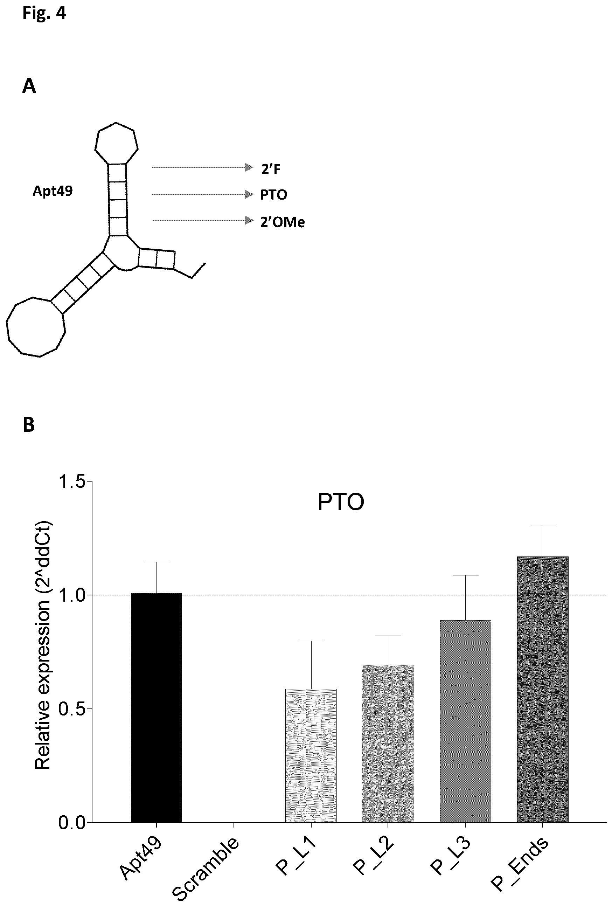

The aptamers of the invention may include chemical modifications in one or more positions as described herein. For illustrative purposes, Apt49 was chemically modified by introducing phosphorothioate (PTO) and 2'-O-methylation (2'OMe) modifications and was shown to maintain its cell internalization properties (see Fig.4A-4D).

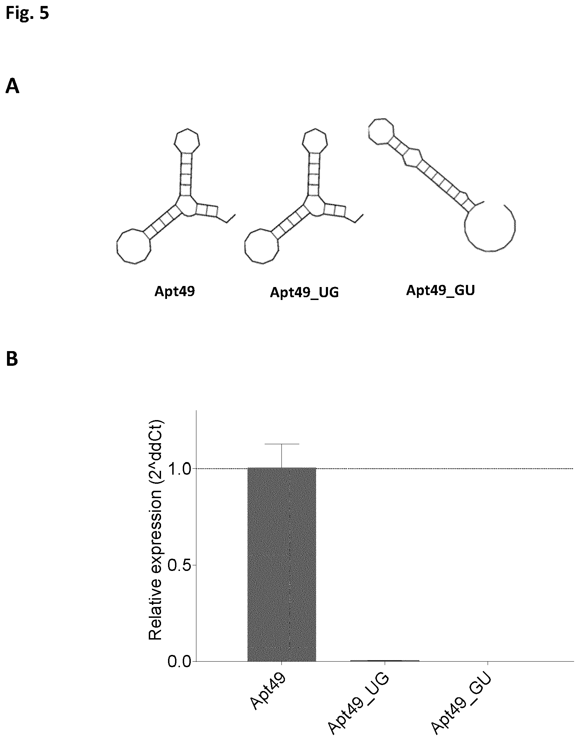

Example 5 emphasizes the criticality of positions 20 and 29 of an aptamer of formula I which were modified with respect to AptP. Indeed, as shown in Fig. 5B mutation of these positions results in a drastic decrease of the aptamer internalization.

Finally, Example 6 shows that 44 of the 56 positive sequences have a 3-stem structure as their predicted most stable fold.

Accordingly, the first aspect of the invention relates to a nucleic acid aptamer that binds specifically to EphA2, preferably human EphA2, the aptamer comprising the three polynucleotide regions Region 1, Region 2, and Region 3 defined below provided in the 5' to 3' order of the aptamer, wherein:
i. Region 1 is of formula (R1):

   5' n₁ n₂ G A G n₆ n₇ C n₉ n₁₀ n₁₁ n₁₂ n₁₃ G G n₁₆ n₁₇ C 3'

   wherein:
   n₁ and n₂ are independently selected from G, dG or - ;
   n₆ is G, dG or-;
   n₇ is A, dA or-;
   n₉ is G, dG or-;
   n₁₀ is A, dA or-;
   n₁₁ is U, dU or-;
   n₁₂ is G, dG or-;
   n₁₃ is C, dC or-;
   n₁₆ is U, dU or-;
   n₁₇ is C, dC or -;
   wherein G, A, U, and C are ribonucleotides; dG, dA, dU, and dC are 2'-deoxyribonucleotides, wherein unless otherwise indicated, the respective nucleotide includes the unmodified nucleotide and modified forms thereof, and "-" means absence of nucleotide;
   provided that
   n₆ and n₁₇ are complementary nucleotides or, alternatively, are absent;
   n₇ and n₁₆ are complementary nucleotides or, alternately, are absent; in preferred embodiments, when n₁ is -, n₁₂ is -; and
   also preferably, when n₇ is -, n₁₁ is U or dU;
ii. Region 2 is of formula (R2):

   5' U G n₂₁ n₂₂ n₂₃ n₂₄ n₂₅ U G n₂₈ U C n₃₁ U C C C C A n₃₈ n₃₉ n₄₀ n₄₁ n₄₂ 3'

   wherein:
   n₂₁ is U, dU or -;
   n₂₂ is C, dC or -;
   n₂₃ is G, dG or -;
   n₂₄ is U, dU or -;
   n₂₅ is C, dC or -;
   n₂₈ is U, dU or -;
   n₃₁ is G, dG or -;
   n₃₈ is G, dG or-;
   n₃₉ is A, dA or -;
   n₄₀ is C, dC or-;
   n₄₁ is G, dG or-;
   n₄₂ is A, dA or-;
   provided that:
   n₂₁ and n₄₂ are complementary nucleotides or, alternatively, are absent;
   n₂₂ and n₄₁ are complementary nucleotides or, alternatively, are absent;
   n₂₃ and n₄₀ are complementary nucleotides or, alternatively, are absent;
   n₂₄ and n₃₉ are complementary nucleotides or, alternatively, are absent; and
   n₂₅ and n₃₈ are complementary nucleotides or, alternatively, are absent;
iii. Region 3 is of formula (R3):

   5' C U C G C n₄₈ n₄₉ n₅₀ n₅₁ 3'

   wherein:
   n₄₈ is C, dC or -;
   n₄₉ is C, dC or -;
   n₅₀ is G, dG or-;
   n₅₁ is A, dA or -;
   provided that
   n₁ of R1 and n₄₉ of R3 are complementary nucleotides or, alternatively, are absent; and
   n₂ of R1 and n₄₈ of R3 are complementary nucleotides or, alternatively, are absent;
   wherein said aptamer is preferably further characterized by:
   a) having a length of 50 nucleotides or less; and/or
   b) n₁₁ and/or n₁₂ of R1 is absent.

In a second aspect, the present invention provides a nucleic acid aptamer that binds specifically to EphA2, preferably human EphA2, wherein said nucleic acid aptamer is selected from the group consisting of the aptamers in the sequences identified in Table 2 .1 and/or 2.2, or a variant having at least 85%, preferably at least 90% identity, more preferably at least 95% identity to any thereof.

In a third aspect, the invention relates to a process for producing a nucleic acid aptamer of the first or second aspect. In some embodiments, said process comprises synthesizing the aptamer by standard solid phase synthesis using the phosphoramidite method. In other embodiments, the nucleic acid aptamer is synthesized by *in vitro* transcription using standard or modified retrotranscriptases or as a direct result of the Systematic Evolution of Ligands by Exponential Enrichment (SELEX) process.

In a fourth aspect, the present invention provides a complex comprising the aptamer as defined in the first or second aspect of the invention and one or more moieties.

In a fifth aspect, the invention relates to the process for producing a complex of the fourth aspect of the invention, wherein said process comprises:
(i) providing a nucleic acid aptamer of the first aspect of the invention; and
(ii) directly or indirectly linking the aptamer to the one or more moieties described herein.

In a sixth aspect, the present invention provides a composition comprising the aptamer as defined in the first or second aspect of the invention or the complex as defined in the fourth aspect of the invention.

In a seventh aspect, the present invention provides the aptamer as defined in the first or second aspect of the invention, the complex as defined in the fourth aspect of the invention, or the composition as defined in the sixth aspect of the invention, for use as a medicament, alone or as a combination therapy.

In an eighth aspect, the present invention provides the use of the aptamer as defined in the first or second aspect of the invention or the complex as defined in the fourth aspect of the invention as EphA2 detection or quantification agent; or as a cellular internalization vehicle targeting cells expressing EphA2.

In a ninth aspect the present invention provides the aptamer as defined in the first or second aspect of the invention, the complex as defined in the fourth aspect of the invention or the composition as defined in the sixth aspect of the invention, for use in a method of *in vivo* detection of a disease; or in a method of *in vivo* detection or quantification of a target protein; or alternatively, in a method comprising *in vivo* detection or quantification of a target protein, such as in a method for the *in vivo* screening diagnosis, prognosis or monitoring of a disease wherein an increase or decrease of the levels of said target protein has been associated with the presence or prognosis of the disease. In some embodiments, the method is for the *in vivo* detection of EphA2 or EphA2-expressing cells, and the method is for the detection of a EphA2-related disease. In preferred embodiments, said EphA2-expressing cells are EphA2-expressing cancer cells and the disease is a EphA2-expressing cancer.

This aspect can alternatively be formulated as a method for the *in vivo* detection of a disease (e.g., screening diagnosis, or monitoring) or the prognosis of a disease, such as a EphA2 related disease in a subject, wherein said disease is characterized by comprising EphA2-expressing cells, the method comprising the steps of: (a) administering an effective amount of the aptamer as defined in the first or second aspect of the invention, the complex as defined in the fourth aspect of the invention or the composition as defined in the sixth aspect of the invention to a subject; (b) *in vivo* determining the amount of EphA2 (e.g., by directly or indirectly detecting the amount of aptamer bound to EphA2); and (c) comparing it with a reference value; wherein if the amount of EphA2 in the sample is increased or decreased, e.g., statistically deviates, from the reference value, this is indicative of the presence of (e.g., the subject suffers from) the EphA2 related disease or a worst prognosis of the EphA2 related disease. In some embodiments, the presence or prognosis of the disease is associated to increased levels of EphA2 (e.g., a cancer characterized by expressing EphA2 as defined herein) and an increase of the amount of EphA2 in the sample with respect to the reference value, is indicative of the presence of (e.g., the subject suffers from) the EphA2 related disease or a worst prognosis of the EphA2 related disease.

In a tenth aspect, the present invention provides the use of the nucleic acid aptamer as defined in the first or second aspect of the invention, the complex as defined in the fourth aspect of the invention, or the composition as defined in the sixth aspect of the invention, as a detection or quantification agent in a method of *in vitro* or ex *vivo* detection of a disease, or in a method of *in vitro* or ex *vivo* detection or quantification of a target protein; or alternatively in a method comprising the *in vitro* or ex *vivo* detection or quantification of a target protein, such as in a method for the *in vitro* or ex *vivo* screening diagnosis, prognosis or monitoring of a disease wherein an increase or decrease of the levels of said target protein has been associated with the presence or prognosis of the disease. In some embodiments, the method is for the *in vitro* or ex *vivo* detection of EphA2 or EphA2-expressing cells, and the method is for the detection of a EphA2-related disease. In preferred embodiments, said EphA2-expressing cells are EphA2-expressing cancer cells and the disease is a EphA2-expressing cancer.

This aspect can alternatively be formulated as a method for the *in vitro* or ex *vivo* detection of a disease (e.g., screening diagnosis, or monitoring) or the prognosis of a disease, such as a EphA2 related disease in a subject, wherein said disease is characterized by comprising EphA2-expressing cells, the method comprising the steps of: (a) contacting the aptamer as defined in the first aspect of the invention, the complex as defined in the fourth aspect of the invention or the composition as defined in the sixth aspect of the invention, with an isolated test sample from the subject; (b) determining the amount of EphA2 in the test sample EphA2 (e.g., by directly or indirectly detecting the amount of aptamer bound to EphA2); and (c) comparing it with a reference value; wherein if the amount of EphA2 in the sample is increased or decreased, e.g., statistically deviates, from the reference value, this is indicative of the presence (e.g., the subject suffers from) the EphA2 related disease or a worst prognosis of the EphA2 related disease. In some embodiments, the presence or prognosis of the disease is associated to increased levels of EphA2 (e.g., a cancer characterized by expressing EphA2 as defined herein) and an increase of the amount of EphA2 in the sample with respect to the reference value, is indicative of the presence (e.g., the subject suffers from) the EphA2 related disease or a worst prognosis of the EphA2 related disease.

In an eleventh aspect, the present invention provides a kit for the detection or quantification of EphA2 comprising the aptamer as defined in the first or second aspect of the invention, the complex as defined in the fourth aspect of the invention; or the composition as defined in the sixth aspect of the invention; and optionally means to detect the aptamer or complex.

In a twelfth aspect, the present invention provides a method for the detection or quantification of EphA2, wherein said method comprises a step of detecting or quantifying EphA2 using the aptamer as defined in the first or second aspect, the complex as defined in the fourth aspect of the invention, the composition as defined in the sixth aspect of the invention, or the kit as defined in the eleventh aspect of the invention.

In a thirteenth aspect, the present invention provides the use of the kit comprising the aptamer, the complex; or the composition as defined in the eleventh aspect of the invention; for the detection or quantification of EphA2,
in a method for the *in vitro or ex vivo* detection or quantification of EphA2, or
in a method comprising the *in vitro or ex vivo* detection or quantification of EphA2 for the screening, diagnosis, prognosis, or monitoring of a EphA2-related disease, such as a EphA2-expressing cancer.

In a fourteenth aspect, the present invention provides the aptamer as defined in the first or second aspect of the invention, the complex as defined in the fourth aspect of the invention or the composition as defined in the sixth aspect of the invention, for use in a method of prophylactically or therapeutically treating cancer or a EphA2 related disease, preferably a EphA2-expressing cancer.

This aspect can also be formulated as the use of the aptamer, complex, or pharmaceutical composition as defined above for the manufacture of a medicament for the treatment of cancer or a EphA2-related disease, preferably a EphA2-expressing cancer. This aspect can also be formulated as a method for the treatment of cancer or a EphA2-related disease, preferably a EphA2-expressing cancer, in a subject, the method comprising administering a therapeutically effective amount of the aptamer, complex or pharmaceutical composition defined above to a subject in need thereof.

### BRIEF DESCRIPTION OF THE DRAWINGS

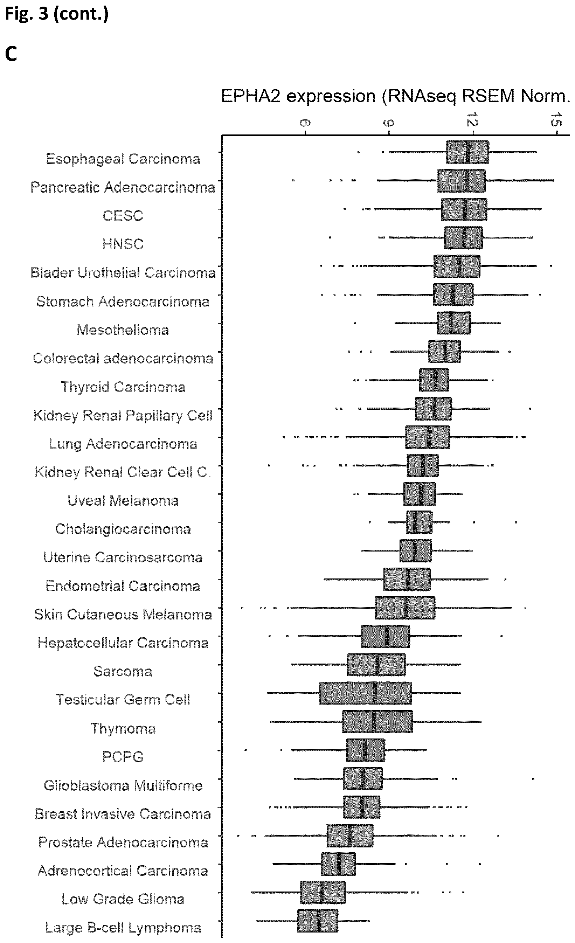

**Figure 1****. *In silico* predicted structures**. A) conformational structure of AptP showing the predicted loops and stems A.1.) generated with RNAfold WebServer and A.2.) generated with Mfold (Gruber AR, Lorenz R, Bernhart SH, Neuböck R, Hofacker IL. The Vienna RNA Websuite. Nucleic Acids Research, Volume 36, Issue suppl_2, 1 July 2008, Pages W70-W74,; Lorenz, R. and Bernhart, S.H. and Höner zu Siederdissen, C. and Tafer, H. and Flamm, C. and Stadler, P.F. and Hofacker, I.L. "ViennaRNA Package 2.0", Algorithms for Molecular Biology, 6:1 page(s): 26, 2011), the predicted structure is a 3 stem conformation (stem 1 in dots, stem 2 in grey and stem 3 in **black**) which also has 2 loops referred as L1 and L2 , respectively as depicted. B) Some examples of *in silico* structures within the group of Apt1 to Apt29. C) Some examples of *in silico* predicted structures within the group of Apt30 to Apt64. D) Three *in silico* structures within the group of Apt65 to Apt108. The underlined aptamers in Fig. 1 B and C were those used in the migration assays shown in Fig. 3. E) Graph showing the aptamer of formula (I) and the corresponding nucleotides at positions 1 to 51.

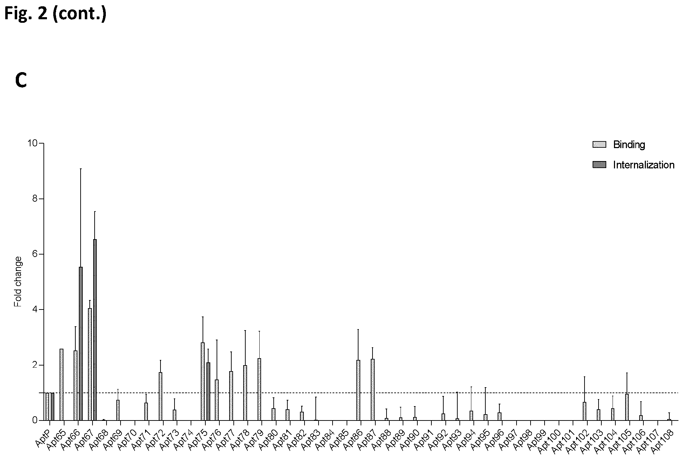

**Figure 2****. Binding and cell internalization results.** A) Binding and *in vitro* Internalization results in A673 cells for Apt1 to Apt29. B) Binding and *in vitro* Internalization results in A673 cells for Apt30 to Apt64. C) Binding and *in vitro* Internalization results in A673 cells for Apt65 to Apt108.
**Figure 3****. Functional assays.** A) Pictures taken with a Nikon600 inverted microscope of the different aptamer treatments in A673 cells. B) Quantification of the migration assay performed with the best candidates selected upon binding and internalization studies. C) EphA2 receptor expression in different cancer types. This figure was prepared by the inventors using data from The Cancer Genome Atlas Program (TCGA) database. D) Aptamer internalization in different EphA2 expressing cell lines.

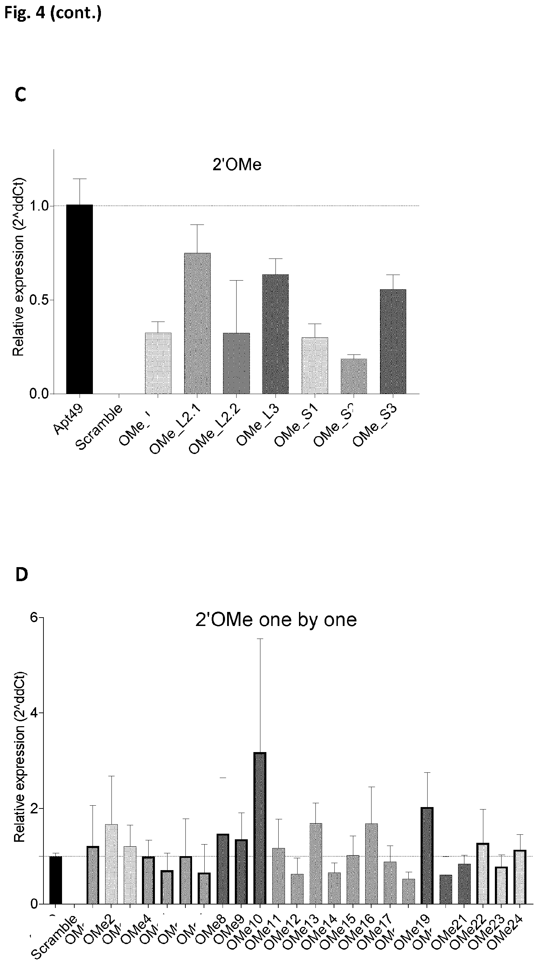

**Figure 4****. Chemical modifications.** A) Apt49 aptamer and its chemical modifications B) Phosphorothioate backbone modifications (PTOs) and their effect on internalization. C) 2'-O-methyl sugar modifications (2' OMes) by blocks and their effect on aptamer internalization. D) 2' OMes modifications performed one by one and their internalization results.
**Figure 5****. Base substitutions and its relevance for aptamer functionality.** Substitution of the nucleotides in positions 20 and 29 of formula I in Apt49 and its effect on A) predicted secondary aptamer structure and B) aptamer internalization.
**Figure 6****. Apt49 predicted folded structure**. Regions of a 3-stem structure are highlighted (legend: stem 1 in dots, stem 2 in grey, stem 3 in black).

### DETAILED DESCRIPTION OF THE INVENTION

The present invention provides in a first aspect a nucleic acid aptamer which binds specifically to EphA2, preferably human EphA2, as defined above.

The term "aptamer" as used herein refers in general to either an oligonucleotide of a single defined sequence or a mixture of said oligonucleotides, wherein the mixture retains the properties of binding specifically to EphA2. As used herein, "aptamer" refers to a single-stranded nucleic acid. Preferably, the aptamers of the present disclosure are structure-based protein binding oligonucleotides.

The term "oligonucleotide" as used herein is generic to polydeoxyribonucleotides (containing 2'-deoxy-D-ribose or modified forms thereof), i.e. DNA, to polyribonucleotides (containing D ribose or modified forms thereof), i.e. RNA, and to any other type of polynucleotide which is an N-glycoside or C-glycoside of a purine or pyrimidine base, or modified purine or pyrimidine base or a basic nucleotide. According to the present disclosure the term "oligonucleotide" includes not only those with conventional bases, sugar residues and internucleotide linkages, but also those that contain modifications of any or all of these three moieties (hereinafter also referred as "modified nucleotides").

The term "base" can be interchangeably used by "nucleotide base", "nucleobase" or "residue". In some embodiments, said aptamer is an RNA aptamer and said nucleotides are ribonucleotides. The term "RNA-aptamer" as used herein is an aptamer comprising ribonucleoside units, such as adenosine, guanosine, 5-methyluridine, uridine, 5-methylcytidine, cytidine, pseudouridine, inosine, N6-methyladenosine, xanthosine, and wybutosine.

In some embodiments, the "nucleotide base" of ribonucleotides is selected from guanine (G), adenine (A), uracil (U) or cytosine (C) and modified versions thereof as described herein below. The term "base" may also be generically used herein to refer to "nucleotide".

An L-nucleic acid as used herein is a nucleic acid or nucleic acid molecule consisting of L-nucleotides, preferably consisting completely of L-nucleotides. A D-nucleic acid as used herein is nucleic acid or nucleic acid molecule consisting of D-nucleotides, preferably consisting completely of D-nucleotides. The terms nucleic acid and nucleic acid molecule are used herein in an interchangeable manner if not explicitly indicated to the contrary. Also, if not indicated to the contrary, any nucleotide sequence is set forth herein in 5' → 3' direction.

Irrespective of whether the nucleic acid molecule of the invention consists of D-nucleotides, L-nucleotides or a combination of both with the combination being e.g. a random combination or a defined sequence of stretches consisting of at least one L-nucleotide and at least one D-nucleic acid, the nucleic acid may consist of desoxyribonucleotide(s), ribonucleotide(s) or combinations thereof. It is also within the present invention that the nucleic acid molecule consists of both ribonucleotides and 2'deoxyribonucleotides. In order to distinguish between ribonucleotides and 2'deoxyribonucleotides in the sequences of the nucleic acid molecules according to the present invention the following reference code is used herein.

The nucleic acid molecule according to the present invention may consist of 2'deoxyribonucleotides, wherein
dG is 2'deoxy-guanosine-5'-monophosphate,
dC is 2'deoxy-cytidine-5'-monophosphate,
dA is 2'deoxy-adenosine-5'-monophosphate,
dT is a 2'deoxy-thymidine-5'-monophosphate, and
dU is 2'deoxy-uridine 5'monophosphate.

The nucleic acid molecule according to the present invention may consist of ribonucleotides, wherein
G is guanosine-5'-monophosphate,
C is cytidine 5'-monophosphate,
A is adenosine-5'-monophosphate,
U is uridine-5'monophosphate, and
rT is methyluridine-5'-monophosphate (ribothymidine monophosphate).

Unless indicated otherwise, the above-mentioned ribonucleotides and deoxyribonucleotides may be the conventional (unmodified) nucleotide or a modified form thereof as described herein.

Complementarity is achieved by distinct interactions between nucleobases: adenine-uracil/thymine, guanine-cytosine. Adenine and guanine are purines, while cytosine, thymine and uracil are pyrimidines. Purines are larger than pyrimidines. Both types of molecules complement each other and can only base pair with the opposing type of nucleobase. In nucleic acids, nucleobases pair with each other by hydrogen bonding, which only works efficiently between adenine and uracil/thymine and between guanine and cytosine.

The following table provides for each of the nucleotides defined herein above (first column) its alternative complementarity nucleotides (second column):

| **Nucleotides** | **Complementary nucleotides** |
|---|---|
| **C** | G; dG |
| **G** | C; dC |
| **A** | U; dU |
| | rT; dT |
| **U** | A; dA |
| **rT** | A; dA |
| **dC** | G; dG |
| **dG** | C; dC |
| **dA** | U; dU |
| | rT; dT |
| **dU** | A; dA |
| **dT** | A; dA |

In preferred embodiments, the complementary nucleotides are as follows:

| **Nucleotides** | **Complementary nucleotides** |
|---|---|
| **C** | G |
| **G** | C |
| **A** | U |
| | rT |
| **U** | A |
| **rT** | A |
| **dC** | dG |
| **dG** | dC |
| **dA** | dU |
| | dT |
| **dU** | dA |
| **dT** | dA |

The term "nucleotide" also includes "modified nucleotide". The term "modified" encompasses nucleotides with a covalently modified base and/or sugar. For example, modified nucleotides include nucleotides having sugars which are covalently attached to low molecular weight organic groups other than a hydroxyl group at the 3' position and other than a phosphate group at the 5' position. Thus, modified nucleotides may also include 2' substituted sugars such as 2'-O-methyl; 2'-O-alkyl; 2'-O-allyl; 2'-S-alkyl; 2'-S-allyl; 2'-fluoro-; 2'-halo or 2'-azido-ribose, carbocyclic sugar analogues, anomeric sugars; epimeric sugars such as arabinose, xyloses or lyxoses, pyranose sugars, furanose sugars, and sedoheptulose. Modified nucleotides are known in the art and include, by example and not by way of limitation, alkylated purines and/or pyrimidines; acyiated purines and/or pyrimidines; or other heterocycles. These classes of pyrimidines and purines are known in the art and include, pseudoisocytosine; N4, N4-ethatiocytosine; 8-hydroxy-N6-methyladenine; 4-acetylcytosine, 5-carboxyhydroxylmethyl uracil; 5-fluorouracil.; 5-bromouracil; 5-carboxymethylaminomethyl-2-thiouracil; 5-carboxymethylaminomethyl uracil; dihydrouracil; inosine; N6-isopentyl-adenine; 1-methyladenine; 1-methylpseudouracil; 1-methylguanine; 2,2-dimethylguanine; 2-methyladenine; 2-methylguanine; 3-methylcytosine: 5-methyleytosine; 6-methyladenine; 7-methylguanine; 5-methylaminomethyl uracil; 5-methoxyaminomethyl-2-thiouracil; β-D-mamiosylqueosine; 5-methoxycarbonilmethyluracil; 5-methoxyuracil; N6-(2-Isopentenyl)adenine; uracil-5-oxyacetic acid methyl ester; pseudouracil; 2-thiocytosine; 5-methyl-2-thiouracil; 2-thiouracil; 4-thiouracil; 5-methyluracil; N-uracil-5-oxyacetic acid methylester; uracil 5-oxyacetic acid; queosine; 2-thiocytosine; 5-propyluracil; 5-propylcytosine; 5-ethyluracil; 5-ethylcytosine; 5-butyluracil; 5-pentyluracil; 5-pentylcytosine; and 2,6,-diaminopurine; methylpsuedouracil; I-methylguanine; 1-methylcytosine.

In some embodiments, one or more of the nucleotides of the nucleic acid aptamer are chemically modified (Odeh F, et al. 2019. Aptamers Chemistry: Chemical Modifications and Conjugation Strategies. Molecules. 2019 Dec 18;25(1):3, Chen Z et al. 2023). In some embodiments, said chemical modification includes one or more of the following, as well as combinations thereof:
i. modifications on the sugar ring, such as 2' -substitutions, 4' -oxygen replacement with a sulfur atom or locked nucleic acids (LNAs),
ii. modifications on the phosphodiester linkage, such as methylphosphonate or phosphorotihioate and triazole modification; and
iii. modifications on the nucleic acid bases, such as on pyrimidines' C5-position and the N7 position of purines.

In other embodiments, aptamer modifications include one or more of the following, as well as combinations thereof: (a) Nucleotides modified by replacing the 2' position with either a fluoro- (F), amino- (NH2) or O-methyl (OCH3) group for enhanced nuclease resistance. These modified nucleotides are introduced either chemically or enzymatically. (b) Bridging phosphorothioates incorporated enzymatically. (c) End caps that involve reversing the polarity of the chain incorporated during chemical synthesis, (d) Linkers inserted at the 5 '-ends of aptamers by either chemical or enzymatic means to provide handles for conjugation or to alter pharmacokinetic properties. Keefe et al, Aptamers as therapeutics, Nat Rev Drug Discov. 2010 Jui;9(7):537-50.

In certain embodiments, modification of the 2'-position of the ribose indirectly improves nuclease resistance of the internucleotide phosphate bond and at the same time increases duplex stability (Tm), and also provides protection from immune activation. 2'-O- methyl RNA (2'OMe) is a naturally occurring RNA variant found in mammalian ribosomal RNAs and transfer RNAs.

In some embodiments, at least one, preferably all, of the pyrimidine moieties of the nucleotide sequence are 2' substituted pyrimidines. For instance, the one or more 2' substitutions are selected from the group consisting of 2'-F (2'- Fluor), 2'-MOE (2'-Methoxyethyl) and 2'-OMe (2'-O-Methyl).

In some embodiments, all the pyrimidines are 2'-F substituted pyrimidines. In other embodiments, the aptamer contains 2'F/2'OMe modifications. The number and order of 2'F and 2'OMe modifications is not particularly limited. For instance, these may be a random combination, alternating or in blocks which can include 2 or more consecutive nucleotides with the same modification. In a preferred embodiment, said aptamer is a modified version of Apt49 (SEQ ID NO: 49), which is characterized by having a 2'F modification in all pyrimidines, wherein the 2'F modification is replaced by a 2'-OMe modification in one or more of positions 2, 9, 10, 13, 16 or 19 of Apt49.

Locked nucleic acids (LNAs) contain a methylene bridge which connects the 2'-0 with the 4'-C of the ribose. The methylene bridge 'locks" the sugar in the 3'-endo conformation, providing both a significant increase in Tm as well as nuclease resistance. In some embodiments, the aptamers of the invention may contain LNAs, such as in hybrid RNA-LNAs oligonucleotides.

As used herein, the term "specifically binds" shall be taken to mean that the aptamer reacts or associates more frequently, more rapidly, with greater duration and/or with greater affinity with a particular cell or substance than it does with alternative cells or substances. For example, an RNA aptamer that specifically binds to a target protein binds that protein or an epitope or immunogenic fragment thereof with greater affinity, avidity, more readily, and/or with greater duration than it binds to unrelated protein and/or epitopes or immunogenic fragments thereof. It is also understood by reading this definition that, for example, an RNA aptamer that specifically binds to a first target may or may not specifically bind to a second target. As such, "specific binding" does not necessarily require exclusive binding or non-detectable binding of another molecule, this is encompassed by the term "selective binding". Generally, but not necessarily, reference to binding means specific binding.

The term "affinity" as used herein may refer to the equilibrium constant for the dissociation of an antigen with an antigen-binding molecule (KD), and is considered a measure for the binding strength between an antigenic determinant and an antigen-binding site on the antigen -binding molecule: the lesser the value of the KD, the stronger the binding strength between an antigenic determinant and the antigen-binding molecule (alternatively, the affinity can also be expressed as the association constant (KA), which is 1/KD). It will be clear to the skilled person that the dissociation constant may be the actual or apparent dissociation constant. The KD can also be expressed as the ratio of the dissociation rate constant of a complex, denoted as koff, to the rate of its association, denoted kon (so that KD =koff/kon and KA = korAoff). The off-rate koff has units s⁻¹ (where s is the SI unit notation of second). The on-rate kon has units M⁻¹s⁻¹. The on-rate may vary between 10² M⁻¹s⁻¹ to about 10⁷ M⁻¹s⁻¹, approaching the diffusion-limited association rate constant for bimolecular interactions. The off-rate is related to the half-life of a given molecular interaction by the relation t_{1/2}=ln(2)/koff. The off -rate may vary between 10⁻⁶ s⁻¹ (near irreversible complex with a t_{1/2} of multiple days) to 1s⁻¹ (t_{1/2}=0.69 s). The affinity of a molecular interaction between two molecules can be measured via different techniques known per se, such as the well the known surface plasmon resonance (SPR) biosensor technique (see for example Ober et ah, Intern. Immunology, 13, 1551-1559, 2001) where one molecule is immobilized on the biosensor chip and the other molecule is passed over the immobilized molecule under flow conditions yielding kon, koff measurements and hence KD (or KA) values. This can for example be performed using the well-known BIACORE instruments.

The term "avidity" as used herein may refer to the measure of the strength of binding between an antigen-binding molecule and the pertinent antigen. Avidity is related to both the affinity between an antigenic determinant and its antigen binding site on the antigen-binding molecule and the number of pertinent binding sites present on the antigen-binding molecule.

The aptamer of the invention is characterized by its capacity to bind EphA2. The capacity of an aptamer to bind to EphA2 can be determined by means of any suitable method which allows determining the binding between two molecules, such as between an affinity reagent (e.g., an antibody or an aptamer) and its target antigen. These methods include, for example, surface plasmon resonance (SPR), or competitive binding assays, such as radioimmunoassays (RIA), enzyme immunoassays (EIA) and sandwich competition assays, and the different variants thereof known per se in the art; as well as the other techniques mentioned herein. A person skilled in the art will understand that a variant of these antibody-based methods can be used to determine the ability of the aptamer of the invention to specifically bind EphA2, wherein the affinity reagent is the aptamer defined herein instead of an antibody.

In one embodiment, the capacity of the aptamer to bind EphA2 is determined by contacting EphA2-expressing cells with the aptamer which has been previously labelled (e.g., by the use of a fluorophore). If the signal (e.g., fluorescence signal) is located within the cell, this would be indicative that the aptamer bound to the EphA2 and was subsequently internalized. In an alternative embodiment, the EphA2-expressing cells are contacted with the aptamer and, after a period of time, it is determined the amount of aptamer (e.g., RNA-aptamer) within the cells by RT-PCR, using primers amplifying the aptamer sequence. A person skilled in the art will know how to design specific primers and probes from the aptamer sequence.

EphA2 (ephrin type-A receptor 2) is a protein that in humans is encoded by the EPHA2 gene. This gene belongs to the ephrin receptor subfamily of the protein-tyrosine kinase family. EPH and EPH-related receptors have been implicated in mediating developmental events, particularly in the nervous system. Receptors in the EPH subfamily typically have a single kinase domain and an extracellular region containing a Cys-rich domain and 2 fibronectin type III repeats. The ephrin receptors are divided into two groups based on the similarity of their extracellular domain sequences and their affinities for binding ephrin-A and ephrin-B ligands. This gene encodes a protein that binds ephrin-A ligands. Uniprot Accession number for human receptor: P29317.

In one embodiment of the first aspect, the aptamer comprises or consists of the sequence of formula (I):

5' n₁ n₂ G A G n₆ n₇ C n₉ n₁₀ n₁₁ n₁₂ n₁₃ G G n₁₆ n₁₇ C U G n₂₁ n₂₂ n₂₃ n₂₄ n₂₅ U G n₂₈ U C n₃₁ U C C C C A n₃₈ n₃₉ n₄₀ n₄₁ n₄₂ C U C G C n₄₈ n₄₉ n₅₀ n₅₁ 3,

wherein nucleotides n₁, n₂, n₆, n₇, n₉ to n₁₃, n₁₆, n₁₇, n₂₁ to n₂₅, n₂₈, n₃₁, n₃₈ to n₄₂ and n₄₈ to n₅₁ are as defined herein above, preferably wherein n₂₅ is C and n₂₈ is U.

In some embodiments of the first aspect of the invention, optionally in combination with any of the above, the nucleic acid aptamer is one, wherein
i. when two or more of n₉ to n₁₃ are absent, then n₆ and n₁₇ are complementary nucleotides and n₇ and n₁₆ are complementary nucleotides; and/or
ii. when only one of ng to n₁₃ is absent, then n₁₁ or n₁₂ is absent.

In some embodiments of the first aspect of the invention, optionally in combination with any of the above, the nucleic acid aptamer is one wherein all the nucleotides from ng to n₁₃ are absent, n₆ is complementary to n₁₇, and n₇ is complementary to n₁₆.

In some embodiments of the first aspect of the invention, optionally in combination with any of the above, n₂₃ and n₄₀ are absent; and
n₂₁ is complementary to n₄₂; and/or
n₂₅ is complementary to n₃₈.

In some embodiments of the first aspect of the invention, optionally in combination with any of the above, the nucleic acid aptamer is one wherein
n₂₂ and n₄₁ are absent;
n₂₃ and n₄₀ are absent; and
n₂₄ and n₃₉ are absent; and
preferably, n₆ and n₁₇ are complementary nucleotides; and
preferably, n₇ and n₁₆ are complementary nucleotides.

These features characterize some of the best performing candidates, such as Apt49, Apt66, and Apt75, which all have been shown to found superior EphA2 binding and cell internalization properties.

In some embodiment of the first aspect of the invention, optionally in combination with any of the above, the nucleic acid aptamer is one wherein n₂₁ is U, n₂₅ is C, n₂₈ is U, n₃₁ is G, n₃₈ is G and n42 is A. These features characterize some of the best performing candidates, such as Apt49, Apt66, Apt75, Apt53, Apt47 and Apt4 which all have shown superior EphA2 binding and cell internalization properties.

In some embodiment of the first aspect of the invention, optionally in combination with any of the above, the nucleic acid aptamer is one wherein
n₁ of R1 and n₄₉ of R3 are absent; and/or
n₂ of R1 and n₄₈ of R3 are absent;
n₅₀ is G and n₅₁ is A.

In some embodiment of the first aspect of the invention, optionally in combination with any of the above, the nucleic acid aptamer is one wherein
n₁ of R1 and n₄₉ of R3 are complementary;
n₂ of R1 and n₄₈ of R3 are complementary; and
n₅₀ is G and n₅₁ is A.

In a second aspect the present invention provides a nucleic acid aptamer which binds specifically to EphA2, preferably human EphA2, wherein said nucleic acid aptamer is selected from the group consisting of the sequences identified in Table 2 .1 and/or 2.2, or a variant having at least 70%, at least 75%, at least 80%, at least 85%, preferably at least 90% identity, more preferably at least 95% identity, even more preferably at least 97% identity to any thereof.

In preferred embodiments, the aptamer is characterized by the presence of a "G"" at the position corresponding to position 20 of the aptamer of formula I and a "U" at the position corresponding to position 29 of the aptamer of formula (I).

As used herein, "sequence identity" or 'identity" in the context of two nucleic acid sequences refers to a specified number of residues in the two sequences that are the same when aligned by sequence comparison algorithms or by visual inspection.

As used herein, "percentage of sequence identity" means the value determined by comparing two optimally aligned sequences, wherein the portion of the polynucleotide sequence may comprise additions or deletions {i.e., gaps) as compared to the reference sequence (which does not comprise additions or deletions) for optimal alignment of the two sequences. The percentage is calculated by determining the number of positions at which the identical nucleic acid base occurs in both sequences to yield the number of matched positions, dividing the number of matched positions by the total number of positions in the window of comparison, and multiplying the result by 100 to yield the percentage of sequence identity.

Sequence identity can be determined in several different manners. To determine sequence identity, sequences can be aligned using various methods and computer programs (e.g., BLAST, T-COFFEE, MUSCLE, MAFFT, etc.), available over the world wide web at sites including ncbi.nlm.nili.gov/BLAST, ebi.ac.uk/Tools/msa/tcoffee/, ebi.ac.uk/Tools/msa/muscle/, mafft.cbrc.jp/alignment/software/. See, e.g., Altschul et al. (1990), J. Mol. Bioi. 215:403-10.

Similarly, in one embodiment of the first aspect of the invention, the nucleic acid aptamer is selected from the group consisting of the sequences identified in Table 2.1 and/or 2.2, or a variant having at least 70%, at least 75%, at least 80%, at least 85%, preferably at least 90% identity, more preferably at least 95% identity, even more preferably at least 97% identity to any thereof.

In another embodiment of the first or second aspect of the invention, the nucleic acid aptamer as defined herein above, is further characterized by when using a software for folding prediction, a 3-stem structure is predicted to represent at least 35% of the folded structure. In preferred embodiments, at least 40%, 45%,50%, 55%, 60%, 65%, 70%, 75%, 80%, 85%, 90%, 95%, 96%, 97%, 98% or at least 99% of the predicted structures is the 3-stem folded structure. In preferred embodiments, the more stable fold of the aptamer of the first or second aspect is the 3-stem structure.

For illustrative purposes, the nucleotide sequence of Apt49 (SEQ ID NO: 49) is provided below wherein the predicted regions of a 3-stem structure are highlighted (legend: stem 1 in *italics and underlined*, stem 2 in **grey**, stem 3 in **black**). The corresponding folded structure is shown in **Fig. 6** (the same highlighting of the stem regions is used in Fig. 6 except for stem 1 which is identified by a dotted line).

**GGG**A*GGAC*GAUCG*GUCC*U**GUCU**GUUCGUCCCC**AGAC**UCG**CCC**GA

Software for folding prediction are well known in the art and include for instance OMP Engine (DNA Software), RNA fold (University of Vienna), OligoAnalyzer Tool (Integrated DNA Techonolgies), mFold (UNAFold web server), MXfold2 (MXfold2 web server), UFold (UFold web server), among others. In preferred embodiments, aptamer folding predictions are carried out using Visual OMP v.7.9.81.0 along with OMP Engine v.4.0.3.0, both from DNA Software (John Santalucia Jr, 2007) as described in the Examples).

In some embodiments of the invention, the aptamer has at least 35 nucleotides, preferably, at least 38 nucleotides. In some embodiments, optionally combined with the latter, the aptamer has 50 or less nucleotides. In some embodiments, the aptamer has from 40 to 50 nucleotides. In other embodiments, the aptamer has from 38 to 50 nucleotides, from 39 to 49 nucleotides, from 40 to 48 nucleotides, from 41 to 47 nucleotides, from 42 to 46 nucleotides or from 43 to 45 nucleotides. In preferred embodiments, the aptamer has from 42 to 50 nucleotides.

In some embodiments of the invention, the aptamer is characterized by having one or more of the following characteristics:
a) a binding response superior to AptP as determined by surface plasmon resonance (SPR) using a Biacore biosensor system;
b) binds to human EphA2 with a KD of 1 x 10⁻⁹ M or less;
c) has internalization properties in EphA2 expressing cells superior to AptP,; and/or
d) reduces *in vitro* migration of EphA2 expressing cells, such as A673 cells, compared to untreated cells or with respect to a control aptamer.

In preferred embodiments, the aptamer is characterized by having at least 2, at least 3 or all four of the above-mentioned characteristics.

In some embodiments, the aptamer is characterized by having a binding response wherein the determined value (e.g., at the end of the dissociation) for the aptamer is at least one-fold higher, more preferably at least 1.5, 2, 3, 4, 5, 6, 7, 8, 9 or at least 10-fold higher than that of AptP. In one embodiment, the binding response is determined by surface plasmon resonance (SPR) using a Biacore biosensor system as described in the examples of this application.

In some embodiments, the aptamer binds to EphA2 with a dissociation constant (KD) equal to or less than 500 pM, 250 pM, 200 pM, 150 pM, 100 pM (10⁻¹⁰ M) 10 pM (10⁻¹¹ M), 1pM (10⁻¹² M), 0.1 pM (10⁻¹³ M), 0.01 pM (10⁻¹⁴ M), or 0.001 pM (10⁻¹⁵ M). The dissociation constant may be measured using techniques known in the art. In one embodiment, the dissociation constant is measured using biolayer interferometry. In another embodiment, is determined by SPR using a Biacore biosensor system as described in the examples of this application.

In some embodiments, the aptamer has cell internalization properties superior to AptP, wherein the determined value for the aptamer is at least one-fold higher, preferably at least 1.5 2, 3, 4, 5, 6, 7, 8, 9 or at least 10-fold higher than that of AptP. In one embodiment, the cell internalization properties are determined by using a labelled (e.g., fluorescently labelled) aptamer. In another embodiment, the cell internalization properties are determined by RT-qPCR cell internalization assay as described in the examples of this application.

In some embodiments, the aptamer reduces reduces *in vitro* migration of EphA2 expressing cells with respect to untreated cells or a control aptamer. In preferred embodiments, the aptamer *in vitro* inhibition or reduction properties superior to AptP, In some instances, the determined value for the aptamer is at least one-fold higher, preferably at least 1.5 2, 3, 4, 5, 6, 7, 8, 9 or at least 10-fold higher than that of AptP Examples of EphA2 expressing cells are defined herein above and include EphA2 expressing cancer cells, such as Ewing sarcoma A673 cells, MDA-MB231 breast Cancer cell line, and RH4 Rhabdomyosarcoma cell line. In one embodiment, *in vitro* migration inhibition or reduction properties are determined by a transwell migration assay as described in the examples of this application. In another embodiment, these are determined by the colony formation assay, such as described in Santana-Viera L. et al. 2023.

In some preferred embodiments, the aptamer is one or more, preferably all, selected from the group consisting of the aptamers shown in Table 2.1 which were shown by the inventors to have a EphA2 binding response superior to AptP, as determined by surface plasmon resonance (SPR) using a Biacore biosensor system.

In other preferred embodiments, the aptamer is one or more, preferably all, selected from the group consisting of the aptamers shown in Table 2.2 which were shown by the inventors to have EphA2 binding and cell internalization properties as determined by RT-qPCR cell internalization assay superior to AptP.

In a third aspect, the invention relates to a process for producing a nucleic acid aptamer of the first or second aspect.

The aptamers of the invention can be synthesized by standard solid phase synthesis using the phosphoramidite method which is known in the art (see for instance, Beaucage, S. L. and Lyer, R. P. 1992)". Linkages between nucleotides may use alternative linking molecules. For example, linking groups of the formula P(0)S, (thioate); P(S)S, (dithioate); P(0)NR'2; P(C) )R'; P(0)OR6; CO; or CONR'2 wherein R is H (or a salt) or alkyl (1 -12C) and R6 is alkyl (1-9C) is joined to adjacent nucleotides through- 0-- or- S- .

Accordingly, in some embodiments, the process for producing a nucleic acid aptamer as described herein comprises synthesizing the aptamer by standard solid phase synthesis using the phosphoramidite method.

In other embodiments, the nucleic acid aptamer is synthesized by *in vitro* transcription using standard or modified retrotranscriptases (Zachary J Kartje, Helen I Janis, Shaoni Mukhopadhyay, Keith T Gagnon Revisiting T7 RNA polymerase transcription in vitro with the Broccoli RNA aptamer as a simplified real-time fluorescent reporter J Biol Chem. 2021 Jan-Jun;296:100175) that allow the incorporation of standard nucleotides or modified nucleotides such as 2'F (Kristina W. Thiel, Luiza I. Hernandez, Justin P. Dassie, William H. Thiel, Xiuying Liu, Katie R. Stockdale, Alissa M. Rothman, Frank J. Hernandez, James O. McNamara and Paloma H. Giangrande Delivery of chemo-sensitizing siRNAs to HER2+-breast cancer cells using RNA aptamers Nucleic Acids Research, 2012, Vol. 40, No. 13 6319-6337) or 2'OMe *(*Paula E Burmeister, Scott D Lewis, Robert F Silva, Jeffrey R Preiss, Lillian R Horwitz, P Shannon Pendergrast, Thomas G McCauley, Jeffrey C Kurz, David M Epstein, Charles Wilson, Anthony D Keefe Direct in vitro selection of a 2'-O-methyl aptamer to VEGF Chem Biol. 2005 Jan;12(1):25-33*)* to the aptamer sequence. In still other embodiments, the nucleic acid aptamer is synthesized as a direct result of the Systematic Evolution of Ligands by Exponential Enrichment (SELEX) process (Michael Kohlberger, Gabriele Gadermaier SELEX: Critical factors and optimization strategies for successful aptamer selection Biotechnol Appl Biochem. 2022;69:1771-1792*.).*

Optionally, said method further comprises isolating and/or purifying the obtained aptamer. In some embodiments, aptamer purification is conducted by chromatography, such as by High-performance liquid chromatography (HPLC) or by polyacrylamide gel purification.

The aptamer of the present invention can be coupled to a functional substance forming a complex (also referred to as chimera hereinafter). Like this, the aptamer not only provides a prophylactic or therapeutic effect but also can act as a delivery agent of the functional substance to EphA2 positive cancer cells. Thus, in a fourth aspect, the invention provides a complex comprising the aptamer as defined in any one of the preceding aspects and embodiments, and one or more moieties.

The coupling between the aptamer and the one or more moieties (e.g., a functional moiety) in the complex can be a covalent bond or a non-covalent bond. The complex of the present invention can be one wherein the aptamer of the present invention and one or more (e.g., 2 or 3) of moieties of the same kind or different kinds are bound together.

Preferably, the one or more moieties are coupled to the 3'-end of the aptamer.

In one embodiment of the fourth aspect of the invention, the aptamer is directly linked to the one or more moieties; or, alternatively, the aptamer is indirectly linked through a linker to one or more moieties.

In a particular embodiment of the complex according to any one of the preceding embodiments, a moiety is coupled to the aptamer by a spacer or linker.

Linkers for conjugation of aptamers to other moieties are well known in the art and may be cleavable and non-cleavable linkers. "Cleavable linkers" include for instance acid-labile linkers such as hydrazone bonds and protease-labile linkers such as valine-citrulline dipeptides. "Non-cleavable linkers" include for instance spacers comprising one or more nucleotides, including non-modified and/or modified nucleotides as described herein, or hydrophilic carbon chains such as Hexa(ethylene glycol) (HEG).

In some embodiments, said spacer or linker comprises 2 or more nucleotides, preferably 2-5 nucleotides, more preferably 2 or 3 nucleotides.

In one embodiment of the complex of the invention, the spacer comprises one or more uracil nucleotides. As defined above, these uracil nucleotides can be modified uracil nucleotides. In a preferred embodiment, the spacer is made of uracil nucleotides. For instance, the spacer consists of 2-5 uracil nucleotides, particularly 2 or 3 uracil nucleotides, such as UU or 2'FU2'FU.

In preferred embodiments, the moiety is a functional moiety. The functional moiety is not particularly limited, as far as it newly adds a certain function to the aptamer of the present invention, or is capable of changing (e.g., improving) a certain characteristic which an aptamer of the present invention can possess. As examples of the functional substance, proteins (such as an enzyme), peptides, amino acids, lipids, sugars, monosaccharides, nucleic acids (e.g., a ribozyme), oligonucleotides (such as siRNA, miRNA, antisense oligonucleotides, gapmers) and nucleotides can be mentioned. As further examples of the functional moiety, affinity substances (e.g., biotin, streptavidin, polynucleotides possessing affinity for target complementary sequence (such as siRNA, microRNA (also referred to as miR, mir, or miRNA), shRNA), antibodies, glutathione Sepharose, histidine), substances for labeling (e.g., fluorescent substances, luminescent substances, radioisotopes), enzymes (e.g., horseradish peroxidase, alkaline phosphatase), drugs (e.g., chemotherapeutic agents such as doxorubicin, gemcitabine, etc.) can be mentioned.

In one embodiment of the fourth aspect of the invention, the moiety is selected from:
(i) an antisense oligonucleotide (ASO), siRNA, microRNA, shRNA or a ribozyme;
(ii) a moiety selected from a polysaccharide, a lipid, such as cholesterol or other lipid moieties, a non-proteinaceous polymer, such as one or more polyethylene glycol (PEG) chains, a polyamine, a peptide, a protein, a small molecule and combinations thereof;
(iii) a label for detection purposes; preferably the label is selected from the group consisting of biotin, an enzyme, prosthetic group, fluorescent material, luminescent material, bioluminescent material, electron dense label, labels for magnetic resonance imaging, radioactive material, and combinations thereof;
(iv) a drug;
(vi) a nanoparticle; and
(vii) combinations of any thereof.

In another embodiment of the fourth aspect of the invention, the moiety is a siRNA, preferably a siRNA which recognizes a specific translocation product characterizing the EphA2 expressing cancer.

In a fifth aspect, the invention relates to the process for producing a complex of the fourth aspect of the invention, wherein said process comprises:
(i) providing a nucleic acid aptamer of the first or second aspect of the invention; and
(ii) directly or indirectly linking the aptamer to the one or more moieties described herein.

When the aptamer and the siRNA are linked by nucleotides (e.g., a UU linker) the complex can be directly synthesized by solid phase synthesis as a single entity. Likewise, incorporation of a Hexa(ethylene glycol) linker between the aptamer and the siRNA can be achieved by solid-phase synthesis using Hexa(ethylene glycol) phosphoramidate (A Jäschke, J P Fürste, E Nordhoff, F Hillenkamp, D Cech, and V A Erdmann Synthesis and properties of oligodeoxyribonucleotide-polyethylene glycol conjugates Nucleic Acids Res. 1994 Nov 11; 22(22): 4810-4817). When an acid-labile linker is used the formation of the hydrazone bond can be achieved by conjugation of an aldehyde and hydrazine (Kölme/, Dominik K.; Kool, Eric T. (2017). "Oximes and Hydrazones in Bioconjugation: Mechanism and Catalysis". Chemical Reviews. 117 (15): 10358-10376*).* When a protease-labile linker is used the aptamer may be modified with a thiol or an alkyne group and the siRNA may be modified with an amino group. Additionally, one of the units (aptamer or siRNA) will be modified with a bivalent linker such as valine-citrulline (Doronina, S. O. et al. Development of potent monoclonal antibody auristatin conjugates for cancer therapy. Nat. Biotechnol. 21, 778-784 (2003*)* bearing a maleimide group (react with thiols) or azido group (react with alkynes). A final coupling reaction is performed between the linker modified unit (aptamer or siRNA) with the other unit.

In a sixth aspect the present invention provides a composition comprising the aptamer or the complex as defined in any of the above aspects and embodiments.

In one embodiment of the sixth aspect of the invention, the composition is a pharmaceutical composition comprising pharmaceutically acceptable excipients and/or carriers.

The expression "excipients and/or carriers" refers to acceptable materials, compositions, or vehicles. Each component must be pharmaceutically acceptable in the sense of being compatible with the other ingredients of the composition. It must also be suitable for use in contact with the tissue or organ of humans and non-human animals without excessive toxicity, irritation, allergic response, immunogenicity or other problems or complications commensurate with a reasonable benefit/risk ratio. Examples of suitable acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical or cosmetical composition, its use is contemplated to be within the scope of this invention.

The formulation of the pharmaceutical compositions described herein may be prepared by any method known or hereafter developed in the art of pharmacology. In general, such preparatory methods include the step of bringing the active ingredient (aptamer or complex) into association with an excipient and/or one or more other accessory ingredients, and then, if necessary and/or desirable, shaping and/or packaging the product into a desired single- or multi-dose unit.

A pharmaceutical composition of the invention may be prepared, packaged, and/or sold in bulk, as a single unit dose, and/or as a plurality of single unit doses. As used herein, a "unit dose" is discrete amount of the pharmaceutical composition comprising a predetermined amount of the active ingredient.

The relative amounts of the active ingredient (aptamer or complex of the invention), the acceptable excipients, and/or any additional ingredients in the composition of the invention will vary, depending upon the identity, size, and/or condition of the subject treated and further depending upon the route by which the composition is to be administered.

Examples of the pharmaceutically acceptable carrier include, but are not limited to, excipients such as sucrose, starch, mannit, sorbit, lactose, glucose, cellulose, talc, calcium phosphate, and calcium carbonate; binders such as cellulose, methylcellulose, hydroxylpropylcellulose, polypropylpyrrolidone, gelatin, gum arabic, polyethylene glycol, sucrose, and starch; disintegrants such as starch, carboxymethylcellulose, hydroxylpropylstarch, sodium-glycol-starch, sodium hydrogen carbonate, calcium phosphate, and calcium citrate; lubricants such as magnesium stearate, Aerosil^{®}, talc, and sodium lauryl sulfate; flavoring agents such as citric acid, menthol, glycyrrhizin-ammonium salt, glycine, and orange powder; preservatives such as sodium benzoate, sodium hydrogen sulfite, methylparaben, and propylparaben; stabilizers such as citric acid, sodium citrate, and acetic acid; suspending agent such as methylcellulose, polyvinylpyrrolidone, and aluminum stearate; dispersant such as surfactants; diluents such as water, saline, and orange juice; base waxes such as cacao butter, polyethylene glycol, and white kerosene; and the like.

The composition of the present invention can be formulated in any form known by the skilled in the art suitable for the desired administration (e.g., oral, parenteral, inhalant).

In a particular embodiment according to any one of the preceding embodiments, the aptamer and/or complex of the composition or medicament of the present invention is/are the active principle(s) of the composition.

The present invention also provides a solid phase carrier having the aptamer or the complex of the present invention immobilized thereon. As examples of the solid phase carrier, a substrate, a resin, a plate (e.g., multiwell plate), a filter, a cartridge, a column, and a porous material can be mentioned. The substrate can be one used in DNA chips, protein chips and the like; for example, nickel-PTFE (polytetrafluoroethylene) substrates, glass substrates, apatite substrates, silicon substrates, alumina substrates and the like, and substrates prepared by coating these substrates with a polymer and the like can be mentioned. As examples of the resin, agarose particles, silica particles, a copolymer of acrylamide and N,N'-methylenebisacrylamide, polystyrene-crosslinked divinylbenzene particles, particles of dextran crosslinked with epichlorohydrin, cellulose fiber, crosslinked polymers of allyldextran and N,N'-methylenebisacrylamide, monodispersed synthetic polymers, monodispersed hydrophilic polymers, Sepharose^{®}, Toyopearl^{®} and the like can be mentioned, and also resins prepared by binding various functional groups to these resins were included. The solid phase carrier of the present invention can be useful in, for example, purifying, detecting and quantifying EphA2. The aptamer or the complex of the present invention can be immobilized onto a solid phase carrier by a method known by the skilled person.

The invention also provides therapeutic and/or prophylactic treatment, detection and/or quantification (e.g., screening, diagnosis, prognosis or monitoring) uses and methods of the nucleic acid aptamer, complex or pharmaceutical composition described herein, particularly for managing cancer or EphA2-related diseases, such as EphA2-expressing cancer in a subject.

As used herein, the term "subject" shall be taken to mean any subject, including a human or non-human subject. The non-human subject may include non-human primates, ungulate (bovines, porches, ovines, caprines, equines, buffalo and bison), canine, feline, lagomorph (rabbits, hares and pikas), rodent (mouse, rat, guinea pig, hamster and gerbil), avian, and fish. Preferably, the subject is a human.

The term "treatment", as used herein, refers to the prophylactic and/or therapeutic treatment.

The term "therapeutic treatment", as used herein, unless otherwise indicated, includes the amelioration, cure, and/or maintenance of a cure (i.e., the prevention or delay of relapse) of a disease or disorder. Treatment after a disorder has started aims to reduce, alleviate, ameliorate or altogether eliminate the disorder, and/or its associated symptoms, to prevent it from becoming worse, to slow the rate of progression, or to prevent the disorder from reoccurring once it has been initially eliminated (i.e., to prevent a relapse). The term "treatment", as used herein, unless otherwise indicated, refers to the act of "treating".

The term "prophylactic treatment" as used herein refers to preventing a pathological state. It is noted that, this term as used herein is not understood to include the term "therapeutic treatment" as defined herein.

The expression "therapeutically effective amount" as used herein refers to an amount that is effective, upon single or multiple dose administration to a subject (such as a human patient) in the prophylactic or therapeutic treatment of a disease, disorder or pathological condition. In some embodiments, it refers to sufficient quantity of RNA aptamer, complex or composition according to the present invention to reduce or inhibit the number of EphA2 expressing cancer cells and/or one or more symptoms of cancer. The skilled person will be aware that such an amount will vary depending upon, for example, the particular subject and/or the type or severity or level of disease. The term is not construed to limit the present disclosure to a specific quantity of nucleic acid aptamer, complex or composition.

EphA2-related diseases include but are not limited to cataract, such as posterior polar cataract, Pendred syndrome and cancer, such as EphA2-expressing cancers.

The terms "cancer" and "cancerous" refer to or describe the physiological condition in mammals that is typically characterized by unregulated cell growth. Included in this definition are benign and malignant cancers or tumors, as well as dormant tumors or micrometastases. Examples of cancer include but are not limited to, carcinoma, lymphoma, blastoma, sarcoma, and leukemia.

The term "tumor" is an abnormal mass of tissue that may be benign, premalignant, or cancerous. Preferably, this "tumor" is a cancerous tumor.

In some embodiments, the nucleic acid aptamer, complex or pharmaceutical composition described herein are for the prophylactic or therapeutic treatment of cancer metastasis.

The term "metastasis" as used herein refers to distant metastasis affecting organs other than the primary tumor site. Metastasis may be defined as the process by which cancer spreads or transfers from the primary site to other regions of the body with the development of a similar cancerous lesion at the new location (see for instance: Chambers AF et al., Nat Rev Cancer 2002; 2: 563-72). For instance, in colorectal cancer, metastasis in another organ (e.g., the liver) typically shows an enteroid adenocarcinoma pattern. A "metastatic" or "metastasizing" cell is typically one that loses adhesive contacts with neighboring cells and migrates via the bloodstream or lymph from the primary site of disease to invade neighboring body structures.

As used herein, the expression "cancer characterised by expressing EphA2" or "EphA2 expressing cancer" refers to a tumor or cancer comprising cells expressing EphA2 (EphA2 positive cells). More particularly, it may refer to a cancer over-expressing EphA2, i.e. with cells over-expressing EphA2. It is well-understood by the skilled person in the art which cancers are embraced by the expression "cancer characterised by expressing EphA2" or "EphA2 expressing cancer" (Zhou Y. et al., "Emerging and Diverse Functions of the EphA2 Noncanonical Pathway in Cancer Progression", Biol. Pharm. Bull. 40, 1616-1624 (2017)).

The term "EphA2+", "EphA2 positive" or "EphA2 expressing cell" as used herein may be used interchangeably. The term encompasses cell surface expression of EphA2 which can be detected by any suitable means.

In the context of the invention, the cancer is characterised by expressing EphA2 (EphA2 expressing cancer). Thus, in a particular embodiment the cancer is a cancer comprising EphA2 positive cell(s). Likewise, in another particular embodiment, the cancer is a cancer overexpressing EphA2. Overexpressing EphA2 means that the expression of EphA2 is at least 2, 3, 4 or 5 times higher than the EphA2 expression in healthy tissues. ]

Preferably, the EphA2 expressing cancer is selected from the group consisting of soft tissue and bone sarcomas, in particular TAS, such as ES, ARMS, SS, Ewing-like sarcomas (CIC-, BCOR- and EWSR1-rearranged with non-ETS genes), DSRCT, MLS; embryonal rhabdomyosarcoma; osteosarcoma; breast cancer, in particular triple negative breast cancer; colorectal cancer; melanoma; renal cell carcinoma; pancreatic cancer; prostate cancer, and combinations thereof. More preferably, the EphA2 expressing cancer is selected from the group consisting of soft tissue and bone sarcoma, in particular TAS, such as ES, ARMS, SS; Ewing-like sarcomas (CIC-, BCOR- and EWSR1-rearranged with non-ETS genes); DSRCT, MLS; osteosarcoma; breast cancer, in particular triple negative breast cancer; colorectal cancer; melanoma; renal cell carcinoma; pancreatic cancer; prostate cancer, and combinations thereof. More particularly, the EphA2 expressing cancer is a TAS, preferably ES, ARMS, SS; Ewing-like sarcoma (e.g., CIC-, BCOR- and EWSR1-rearranged with non-ETS genes), DSRCT, MLS, or breast cancer, preferably triple negative breast cancer. Even more preferably the cancer is ES, ARMS or SS.

In other preferred embodiments, the cancer is selected from the group consisting of Ewing sarcoma, Ewing-like sarcomas, rhabdomyosarcoma, such as alveolar rhabdomyosarcoma, colon cancer, prostate cancer, breast cancer, ovarian cancer, bladder cancer, esophageal carcinoma, pancreatic adenocarcinoma, brain cancer, head and neck cancer, cervical squamous cell carcinoma and endocervical adenocarcinoma. All these cancers express EphA2 based on the information available at the TCGA database, see for instance Fig.3C of the Examples.

The dosage of administration of the aptamer, complex, or composition of the present invention varies depending on the kind and activity of active ingredient, seriousness of disease, subject of administration, drug tolerability of the subject of administration, body weight, age and the like, and the usual dosage, based on the amount of active ingredient per day for an adult, can be about 0.0001 to about 100 mg/kg, for example, about 0.0001 to about 10 mg/kg, preferably about 0.005 to about 1 mg/kg.

The nucleic acid aptamer, complex or pharmaceutical composition as defined in any of the above aspects and embodiments can be administered in combination with another drug in the treatment of cancer. In this embodiment, the administration of another drug can be done sequentially or simultaneously. In another embodiment, the administration is made in the form of separate compositions or forming part of the same composition.

The term "another drug" in the context of the invention can preferably be selected from anti-cancer agents, anti-allergic agents, anti-nausea agents (or anti-emetics), pain relievers, cytoprotective agents, and combinations thereof.

In some embodiments, the nucleic acid aptamer, complex or pharmaceutical composition as defined in any of the above aspects and embodiments can be administered in combination with another anti-cancer treatment.

The term "anti-cancer treatment" as used herein may include any treatment to stop or prevent cancer, including but not limited to surgery, radiotherapy, anti-cancer agents and any other existing therapies or to be developed.

The term "anti-cancer agent" as used herein refers to any therapeutic agents useful in treating cancer. Examples of anti-cancer therapeutic agents include, but are limited to, e.g., an inhibitor of the TGFβ signaling pathway, chemotherapeutic agents, growth inhibitory agents, cytotoxic agents, anti-hormonal agents, agents used in radiation therapy, anti-angiogenesis agents, apoptotic agents, anti-tubulin agents, and other agents to treat cancer, such as anti-HER-2 antibodies (e.g., Herceptin^{®}), anti-CD20 antibodies, an epidermal growth factor receptor (EGFR) antagonist (e.g., a tyrosine kinase inhibitor), HER1/EGFR inhibitor (e.g., erlotinib (Tarceva<^{®}>)), platelet derived growth factor inhibitors (e.g., Gleevec^{™} (Imatinib Mesylate)), a COX-2 inhibitor (e.g., celecoxib), interferons, cytokines, antagonists (e.g., neutralizing antibodies) that bind to one or more of the following targets ErbB2, ErbB3, ErbB4, PDGFR-beta, BlyS, APRIL, BCMA or VEGF receptor(s), TRAIL/Apo2, and other bioactive and organic chemical agents, etc. Combinations thereof are also included in the invention.

In a seventh aspect, the present invention provides the aptamer as defined in the first or second aspect of the invention, the complex as defined in the fourth aspect of the invention, or the composition as defined in the sixth aspect of the invention, for use as a medicament, alone or as a combination therapy.

In an eighth aspect, the present invention provides the use of the aptamer as defined in the first or second aspect of the invention or the complex as defined in the fourth aspect of the invention as EphA2 detection or quantification agent; or as a cellular internalization vehicle targeting cells expressing EphA2.

In a ninth aspect the present invention provides the aptamer as defined in the first or second aspect of the invention, the complex as defined in the fourth aspect of the invention or the composition as defined in the sixth aspect of the invention, for use in a method of *in vivo* detection of a disease; or in a method of *in vivo* detection or quantification of a target protein; or alternatively, in a method comprising *in vivo* detection or quantification of a target protein, such as in a method for the *in vivo* screening diagnosis, prognosis or monitoring of a disease wherein an increase or decrease of the levels of said target protein has been associated with the presence or prognosis of the disease. In some embodiments, the method is for the *in vivo* detection of EphA2 or EphA2-expressing cells, and the method is for the detection of a EphA2-related disease. In preferred embodiments, said EphA2-expressing cells are EphA2-expressing cancer cells and the disease is a EphA2-expressing cancer.

In some embodiments, the aptamer as defined in the first or second aspect of the invention, or the complex as defined in the fourth aspect of the invention is labeled for *in vivo* imaging. In one particular embodiment, the aptamer or complex is labeled with a radiotracer for Nuclear Imaging. In another embodiment, the aptamer or complex is labeled with a magnetic contrast agent for Magnetic Resonance Imaging, such as a superparamagnetic contrast agent or a paramagnetic contrast agent.

The term "imaging" or "medical imaging" refers to any technique, method or process used to produce images of the human body for medical purposes. Preferred medical imaging techniques include Magnetic Resonance Imaging and Nuclear Imaging. Nuclear imaging methods typically use the properties of isotopes to visualize labeled molecules bound to the target cells or tissue. Suitable nuclear imaging methods include scintigraphy, PET (positron emission tomography) and SPECT (Single Photon Emission Computed Tomography.

This aspect can alternatively be formulated as a method for the *in vivo* detection of a disease (e.g., screening diagnosis, or monitoring) or the prognosis of a disease, such as a EphA2 related disease in a subject, wherein said disease is characterized by comprising EphA2-expressing cells, the method comprising the steps of: (a) administering an effective amount of the aptamer as defined in the first or second aspect of the invention, the complex as defined in the fourth aspect of the invention or the composition as defined in the sixth aspect of the invention to a subject; (b) *in vivo* determining the amount of EphA2 (e.g., by directly or indirectly detecting the amount of aptamer bound to EphA2); and (c) comparing it with a reference value; wherein if the amount of EphA2 in the sample is increased or decreased, e.g., statistically deviates, from the reference value, this is indicative of the presence of (e.g., the subject suffers from) the EphA2 related disease or a worst prognosis of the EphA2 related disease. In some embodiments, the presence or prognosis of the disease is associated to increased levels of EphA2 (e.g., a cancer characterized by expressing EphA2 as defined herein) and an increase of the amount of EphA2 in the sample with respect to the reference value, is indicative of the presence of (e.g., the subject suffers from) the EphA2 related disease or a worst prognosis of the EphA2 related disease.

The term "reference value", as used herein, relates to a predetermined criteria used as a reference for evaluating the values or data obtained from the samples collected from a subject. This "reference value" may also be referred as "cut-off value" or "threshold value".

The reference value can be an absolute value, a relative value, a value that has an upper or a lower limit, a range of values, an average value, a median value, a mean value, a z-score (e.g. mean value + or- 1 standard deviation (SD)), a tertile value, or a value as compared to a particular control or baseline value.

In addition, it is further noted that a variety of statistical and mathematical methods for establishing the threshold or cut-off level of expression are known in the prior art. A threshold or cut-off expression level for a particular biomarker may be selected, for example, based on data from Receiver Operating Characteristic (ROC) plots. One of skill in the art will appreciate that these threshold or cut-off expression levels can be varied, for example, by moving along the ROC plot for a particular biomarker or combinations thereof, to obtain different values for sensitivity or specificity thereby affecting overall assay performance.

Sensitivity, specificity, and/or accuracy are parameters typically used to describe the validity or performance of a test. In particular, they are used to quantify how good and reliable the discrimination method is. A test is usually calibrated in terms of the desired specificity and sensitivity according to the target use of the test in clinical practice. High sensitivity corresponds to high negative predictive value and is considered generally a desired property for a "rule out" test, such as a screening test which typically will be followed by a confirmatory test. High specificity corresponds to high positive predictive value and is considered generally a desired property for a "rule in" test, such as a companion diagnostic test.

A reference value can be based on an individual sample value but is generally based on a large number of samples, including or excluding the sample to be tested. For instance, this reference value may be derived from a collection of tumor tissue samples from a reference cancer patients' population for whom historical information relating to the actual clinical outcome for the corresponding cancer patient is available.

In the methods of the invention, the value determined in step b) is considered "decreased" when said value is lower than a reference value. Preferably, the value is considered to be lower than a reference value when it is at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than the reference value.

Likewise, in the context of the methods of the invention, the value determined in step b) is considered "increased" when said value is higher than a reference value. Preferably, the value is considered to be higher than a reference value when it is at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than a reference value.

Alternatively or in addition, subjects having more than about 1.1,1.2, 1.3, 1.4, 1.5, 2, 3, 4, 5, 6, 7, 8, 9, 10, 15 or 20 fold levels deviation (i.e., increase or decrease) than the reference value as described herein.

In an tenth aspect, the present invention provides the use of the nucleic acid aptamer as defined in the first or second aspect of the invention, the complex as defined in the fourth aspect of the invention, or the composition as defined in the sixth aspect of the invention, as a detection or quantification agent in a method of *in vitro* or ex *vivo* detection of a disease, or in a method of *in vitro* or ex *vivo* detection or quantification of a target protein; or alternatively in a method comprising the *in vitro* or ex *vivo* detection or quantification of a target protein, such as in a method for the *in vitro* or ex *vivo* screening diagnosis, prognosis or monitoring of a disease wherein an increase or decrease of the levels of said target protein has been associated with the presence or prognosis of the disease.. In some embodiments, the method is for the *in vitro* or ex *vivo* detection of EphA2 or EphA2-expressing cells, and the method is for the detection of a EphA2-related disease. In preferred embodiments, said EphA2-expressing cells are EphA2-expressing cancer cells and the disease is a EphA2-expressing cancer.

This aspect can alternatively be formulated as a method for the *in vitro* or ex *vivo* detection of a disease (e.g., screening diagnosis, or monitoring) or the prognosis of a disease, such as a EphA2 related disease in a subject, wherein said disease is characterized by comprising EphA2-expressing cells, the method comprising the steps of: (a) contacting the aptamer as defined in the first aspect of the invention, the complex as defined in the fourth aspect of the invention or the composition as defined in the sixth aspect of the invention, with an isolated test sample from the subject; (b) determining the amount of EphA2 in the test sample EphA2 (e.g., by directly or indirectly detecting the amount of aptamer bound to EphA2); and (c) comparing with a reference value; wherein if the amount of EphA2 in the sample is increased or decreased, e.g., statistically deviates, from the reference value, this is indicative of the presence (e.g., the subject suffers from) a EphA2 related disease or a worst prognosis of the EphA2 related disease. In some embodiments, the presence or prognosis of the disease is associated to increased levels of EphA2 (e.g., a cancer characterized by expressing EphA2 as defined herein) and an increase of the amount of EphA2 in the sample with respect to the reference value, is indicative of the presence of (e.g., the subject suffers from) the EphA2 related disease or a worst prognosis of the EphA2 related disease.

The *in vitro* methods of the invention can be applied to any type of biological sample from a patient, such as a biopsy sample, tissue, cell or fluid (serum, saliva, semen, sputum, cerebral spinal fluid (CSF), tears, mucus, sweat, milk, brain extracts and the like). In performing the method of the present invention, said biological sample from the cancer patient is preferably a sample containing tumor cells. Tumors or portions thereof may be surgically resected from the patient or obtained by routine biopsy. Preferably, a tumor sample is obtained from the primary tumor. In a particular embodiment, optionally in combination with any of the features or embodiments described above or below, said biological sample isolated from the subject is a tumor biopsy sample, preferably obtained from a resected tumor.

These types of samples are routinely used in the clinical practice and a person skilled in the art will know how to identify the most appropriate means for their obtaining and preservation. Once a sample has been obtained, it may be used fresh, it may be frozen or preserved using appropriate means (e.g., as a formalin-fixed, paraffin-embedded tissue sample). Such biological samples can be taken around the time of diagnosis, before, during or after treatment (e.g. surgical resection).

Methods for quantifying the expression of a specific protein, such as EphA2, are well known in the art. Some well-known methods for determining protein expression levels in a test sample are based in the specific binding of an affinity reagent to a target protein or to a fragment thereof, such as immunoassays or immunohistochemistry (HIC) analysis.

Various types of immunoassays are known to one skilled in the art for the quantitation of proteins of interest. Said immunoassay may be for example an enzyme-linked immunosorbent assay (ELISA), a fluorescent immunosorbent assay (FIA), a chemiluminescence immunoassay (CIA), or a radioimmunoassay (RIA), an enzyme multiplied immunoassay, a solid phase radioimmunoassay (SPROA), a fluorescence polarization (FP) assay, a fluorescence resonance energy transfer (FRET) assay, a time-resolved fluorescence resonance energy transfer (TR-FRET) assay, a surface plasmon resonance (SPR) assay or an immunoblot assay, such as a Western Blot. Multiplex and any next generation versions of any of the above, such as bead-based flow-cytometry immunoassays (e.g., based on the Luminex xMAP technology) are specifically encompassed. In a particular embodiment, said immunoassay is an ELISA assay or any multiplex version thereof.

For determining protein expression and location, immunohistochemical and in-situ hybridization analysis are usually preferred. Immunohistochemistry (IHC) analysis is typically conducted using thin sections of the biological sample immobilized on coated slides. These sections, when derived from paraffin-embedded tissue samples, are deparaffinised and preferably treated so as to retrieve the antigen. The detection can be carried out in individual samples or in tissue microarrays. This procedure, although is subjectively determined by the pathologist, is the standard method of measurement of IHC results, and well known in the art.

The methods of the invention are a variant of the antibody-based methods described herein above, wherein the affinity reagent (i.e., the affinity reagent having specificity against EphA2) is an aptamer of the first or second aspect of the inventor or a complex of the fourth aspect of the invention which is characterized by binding the EphA2 protein or to a fragment thereof, wherein said affinity reagent is preferably labeled. Illustrative, but non-exclusive, examples of labels that can be used include radioactive isotopes, enzymes, fluorophores, chemoluminescent reagents, enzyme cofactors or substrates, enzyme inhibitors, particles, dyes, etc.

In an eleventh aspect, the present invention provides a kit for the detection or quantification of EphA2 comprising the aptamer as defined in the first or second aspect of the invention, the complex as defined in the fourth aspect of the invention; or the composition as defined in the sixth aspect of the invention; and optionally means to detect the aptamer or complex.

In a twelfth aspect, the present invention provides a method for the detection or quantification of EphA2, wherein said method comprises a step of detecting or quantifying EphA2 using the aptamer as defined in the first or second aspect, the complex as defined in the fourth aspect of the invention, the composition as defined in the sixth aspect of the invention, or the kit as defined in the eleventh aspect of the invention.

In a thirteenth aspect, the present invention provides the use of the kit comprising the aptamer, the complex; or the composition as defined in the eleventh aspect of the invention; for the detection or quantification of EphA2, in a method for the *in vitro or ex vivo* detection or quantification of EphA2, or in a method comprising the *in vitro or ex vivo* detection or quantification of EphA2 for the screening, diagnosis, prognosis, or monitoring of EphA2-related diseases, such as a EphA2-expressing cancer.

In a fourteenth aspect, the present invention provides the aptamer as defined in the first or second aspect of the invention, the complex as defined in the fourth aspect of the invention or the composition as defined in the sixth aspect of the invention, for use in a method of prophylactically or therapeutically treating cancer or a EphA2 related disease, preferably a EphA2-expressing cancer.

This aspect can also be formulated as the use of the aptamer, complex, or pharmaceutical composition as defined above for the manufacture of a medicament for the treatment of cancer or a EphA2-related disease, preferably a EphA2-expressing cancer. This aspect can also be formulated as a method for the treatment of cancer or a EphA2-related disease, preferably a EphA2-expressing cancer, in a subject, the method comprising administering a therapeutically effective amount of the aptamer, complex or pharmaceutical composition defined above to a subject in need thereof.

It is contemplated that any features described herein can optionally be combined with any of the embodiments of any aptamer, complex, composition, pharmaceutical composition, methods of producing any thereof; kit, medical use, method of treatment, method of manufacturing a medicament and combination therapies of the invention; and any embodiment discussed in this specification can be implemented with respect to any of these. It will be understood that particular embodiments described herein are shown by way of illustration and not as limitations of the invention.

All publications and patent applications are herein incorporated by reference to the same extent as if each individual publication or patent application were specifically and individually indicated to be incorporated by reference.

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of" excludes any element, step, or ingredient not specified in the claim except for, e.g., impurities ordinarily associated with the element or limitation.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, or 10%. Accordingly, the term "about" may mean the indicated value ± 5% of its value, preferably the indicated value ± 2% of its value, most preferably the term "about" means exactly the indicated value (± 0%). The following examples serve to illustrate the present invention and should not be construed as limiting the scope thereof.

While the foregoing invention has been described in some detail for purposes of clarity and understanding, it will be appreciated by one skilled in the art from a reading of this invention that various changes in form and detail can be made without departing from the true scope of the invention and appended claims.

The examples below serve to further illustrate the invention and are not intended to limit the scope of the present invention.

### EXAMPLES

### Example 1.- Material and Methods

### Cell culture and transfection

Ewing sarcoma cell lines: A673 (acquired from ATCC), A673 EphA2 Control and KO and EW7. MDA-MB231, a Breast Cancer cell line, and RH4, Rhabdomyosarcoma cell line, were also used. Cell lines were cultured in DMEM-F12 (Life Technologies, Carlsbad, California, USA) supplemented with 10% heat-inactivated fetal bovine serum (FBS, Life Technologies) and 1% penicillin-streptomycin (Life Technologies). All cell lines were incubated at 37°C in a humidified atmosphere of 5% CO2 in air and checked regularly for mycoplasma infection. Exponentially growing cells at low passage (lower than 10) were used for all experiments. In the case cells at higher passage than 10 were used, STR analysis was done for cell line authentication.

For transient gene silencing, cells were transfected with Lipofectamine 2000 (Life Technologies) using 1, 2.5, 5, 10 and 12.5 nM of customized siRNA for EWS/Fli-1.

### Binding assay - Aptamer Ranking Measurements

The surface plasmon resonance (SPR) experiments were performed using a Biacore 2000 instrument equipped with a C1 sensor chip (Cytiva). The recombinant human EphA2 receptor (57 kDa, R&D systems) was immobilized using amine-coupling chemistry. In brief, after the chip has been equilibrated with 10 mM Hepes, 150 mM NaCl, pH 7.4, the surfaces of the flow cells were activated for 10 min with a 1:1 mixture of 0.1 M NHS (N-hydroxysuccinimide) and 0.4 M EDC (3-(N,N-dimethylamino) propyl-N-ethylcarbodiimide) at a flow rate of 10 µl/min and at a temperature of 37 °C. The EphA2 receptor at a concentration of 90 nM in 10 mM sodium acetate, pH 4.0, was immobilized at a density of approx. 200 - 500 RU on a measurement flow cell. On a reference flow cell 600 nM bovine serum albumin (66.5 kDa) in 10 mM sodium acetate, pH 5.0, was immobilized at a comparable density. The flow cells were blocked with a 7 min injection of 1 M ethanolamine, pH 8.0.

To rank the aptamers regarding their binding response to the immobilized EphA2 receptor, the aptamers at concentrations of 2.5 nM, 5 nM, 10 nM, 20 nM, or 30 nM were injected over the measurement and the reference flow cell at a flow rate of 30 µl/min and at a temperature of 37 °C. The measurement buffer was 20 mM Hepes, 150 mM NaCl, 2 mM CaCl2, pH 7.4 supplemented with 0.01 % Tween20. The binding complexes were allowed to associate for 60 sec and to dissociate for 90 sec. Subsequently, the flow cells were regenerated with 1 M NaCl for 30 sec. The plotted values are the triple-referenced binding responses recorded after 80 sec of dissociation: Referencing comprises the subtraction of the response of an aptamer on the reference flow cell (first referencing) and the response of the buffer itself from the response of the aptamer on the measurement flow cell (second referencing). For better comparability between the different measurement runs, the double-referenced binding responses were referenced to that of AptP, which served as reference aptamer in each run and was set to 100 % binding response (triple-referenced).

### SPR Kinetic Measurements

The surface plasmon resonance experiments were performed using a Biacore 2000 instrument equipped with a C1 sensor chip (Cytiva). The recombinant human EphA2 receptor (57 kDa, R&D systems) was immobilized using amine-coupling chemistry. Thus, after the chip has been equilibrated with 10 mM Hepes, 150 mM NaCl, pH 7.4 the surfaces of the flow cells were activated for 10 min with a 1:1 mixture of 0.1 M NHS (N-hydroxysuccinimide) and 0.4 M EDC (3-(N,N-dimethylamino) propyl-N-ethylcarbodiimide) at a flow rate of 10 µl/min and at a temperature of 37 °C. The EphA2 receptor at a concentration of 88 nM in 10 mM sodium acetate, pH 4.0, was immobilized at a density of 200-500 RU. On a reference flow cell 600 nM bovine serum albumin (66.5 kDa) in 10 mM sodium acetate, pH 5.0, was immobilized at an almost comparable density. Both flow cells were blocked with a 7 min injection of 1 M ethanolamine, pH 8.0. To collect kinetic binding data, concentrations series of the aptamers in 20 mM Hepes, 150 mM NaCl, 2 mM CaCl₂, pH7.4 supplemented with 0.01 % Tween20, were injected over the two flow cells at a flow rate of 30 µl/min and at a temperature of 37 °C. The complex was allowed to associate and dissociate for 120 s and 360 s, respectively. The surfaces were regenerated with 1 M NaCl for 45 sec. After subtracting the response of the aptamers on the reference surface and the response of the buffer itself from the binding response (double referencing), the resulting binding curves were fitted to a Langmuir 1:1 stoichiometric binding model to determine the kinetic rate constants (kₐ, k_{d}) as well the affinity constant K_{d}. Data analysis was done using the global data analysis option available within BIAevaluation 4.1 software. Two to four independent measurements per aptamer were performed and the geometric means of the determined constants (kₐ, k_{d}, K_{d}) were calculated for each aptamer using GraphPad Prism. *For the exemplary binding curves the following conc. series were injected.*
***Apt49:** 0.47 nM, 0.94 nM, 1.88 nM,* 3.75 *nM, 7.5 nM, 15 nM, 30 nM*
***Apt54:** 0.37 nM, 1.1 nM, 3.3 nM, 10 nM, 30 nM*
***Apt60:** 0.195 nM, 039 nM, 0.78 nM, 1.56 nM, 3.13 nM,* 6.25 *nM, 12.5 nM, 25 nM nM*

### Internalization assay

A673 cells were used for these assays. 200.000 cells were seeded in 6 well-plates and they were cultured for 24 hrs. Cells were first washed with PBS 1X or serum-free medium. Afterwards, cells were incubated with 10 mg/ml of tRNA for 15min at 37°C. Then, 200nM of the aptamers were added to the medium, and they were incubated for 1 hr at 37°C. Swirling every 30 min is recommended. After 1hr, cells were washed once with cold PBS 1X. Then, cells were washed twice with high salt buffer (NaCl 0.5M) and left at 4°C for 5 min. Finally, cells were washed once with PBS 1X prior to lysing the cells for sample obtention. Aptamer quantification was conducted by RT-qPCR as described below.

### Transwell migration assay

Cells were harvested as usual. After an additional wash with DMEM, 250.000 cells in 200µL serum-free medium were added to the top chamber of 8-µm pore polycarbonate transwells (Transwell Permeable Supports - Corning, Corning, New York, USA). Meanwhile, in the bottom chamber, 500 µL of complete medium (10% FBS) were added. For the migration assays in the presence of Aptamers, cells were pre-treated with 100 nM of the candidates 6 hr prior to trypsinization and seeding into the chambers. In addition, the aptamers were also added to the upper chamber. After 24 hr for A673, cells on the upper chamber were removed with a cotton swab. Migrating cells still attached on the membrane's underside were fixed for 30 min using 70% ethanol and stained with crystal violet for 10 min. Transwell membranes were collected and 5 pictures of each transwell were acquired by optical microscopy.

Generally, membranes were discolored with a 100% glacial acetic acid solution and crystal violet was quantified by spectrometry. In some instances, we opted for a direct manual counting of the number of migrating cells in the membrane using imaged. Results are presented as the percentage of a designated control condition.

### RNA extraction and reverse transcription-PCR (RT-PCR)

Total RNA (0.5-1 µg) was extracted by using the Maxwell Instrument from Promega or the NucleoSpin miRNA (for Microarray purpose) from Macherey-Nagel, Duren, Germany, was used for cDNA synthesis with MuLV Reverse Transcriptase (Life Technologies).

### Quantitative real-time PCR

Quantitative reverse transcription-PCR (qRT-PCR) was performed under universal cycling conditions on LightCycler 480 II instrument (Roche) using TaqMan PCR Mastermix and TaqMan probes and small custom TaqMan probes from Life Technologies. Cycle threshold (CT) values were normalized to that of TBP or their specific endogenous control. Relative expression level of the target gene among the different samples was calculated using the ΛΛCT method.

### Aptamers' synthesis

The synthesis of the aptamers was conducted by solid phase chemical synthesis using phosphoroamidites and followed by HPLC purification (Beaucage, S. L.; Lyer, R. P. 1992). These aptamers are 2'F-modified in the pyrimidines (Chen Z et al. 2023).

**Table 1. Aptamer sequences**

| **ID** | **SEQ ID N°:** | **Sequence** | **nts** |
|---|---|---|---|
| Apt1 | 1 | GGGAGGACGAUGCGGUCCUGCGUCUGUUCGUCCCCAGACGCUCGCCCGA | 49 |
| Apt2 | 2 | GGGAGGACGAUGCGGUCCUGCGCUGUUCGUCCCCAGCGCUCGCCCGA | 47 |
| Apt3 | 3 | GGGAGGACGAUGCGGUCCUGCGUCGUUCGUCCCCGACGCUCGCCCGA | 47 |
| Apt4 | 4 | GGGAGGACGAUGCGGUCCUGUCCUGUUCGUCCCCAGGACUCGCCCGA | 47 |
| Apt5 | 5 | GGGAGGACGAUGCGGUCCUGCGCGUUCGUCCCCGCGCUCGCCCGA | 45 |
| Apt6 | 6 | GGGAGGACGAUGCGGUCCUGUCUGUUCGUCCCCAGACUCGCCCGA | 45 |
| Apt7 | 7 | GGGAGGACGAUGCGGUCCUGUCGUCUGUUCGUCCCCAGACGACUCGCCC | 49 |
| Apt8 | 8 | AGGACGAUGCGGUCCUGUCGUCUGUUCGUCCCCAGACGACUCG | 43 |
| Apt9 | 9 | GGACGAUGCGGUCCUGUCGUCUGUUCGUCCCCAGACGACUCG | 42 |
| Apt10 | 10 | CGGACGAUGCGGUCCGUGUCGUCUGUUCGUCCCCAGACGACUCG | 44 |
| Apt11 | 11 | GGGAGGACAUGCGGUCCUGUCGUCUGUUCGUCCCCAGACGACUCGCCCGA | 50 |
| Apt12 | 12 | GGGAGGACGUGCGGUCCUGUCGUCUGUUCGUCCCCAGACGACUCGCCCGA | 50 |
| Apt13 | 13 | GGGAGGACGAGCGGUCCUGUCGUCUGUUCGUCCCCAGACGACUCGCCCGA | 50 |
| Apt14 | 14 | GGGAGGACGAUCGGUCCUGUCGUCUGUUCGUCCCCAGACGACUCGCCCGA | 50 |
| Apt15 | 15 | GGGAGGACGAUGGGUCCUGUCGUCUGUUCGUCCCCAGACGACUCGCCCGA | 50 |
| Apt16 | 16 | GGGAGGACGAUGCGGUCCUGUCGCUGUUCGUCCCCAGCGACUCGCCCGA | 49 |
| Apt17 | 17 | GGGAGGACGAUGCGUCCUGUCGUCUGUUCGUCCCCAGACGACUCGCCCGA | 50 |
| Apt18 | 18 | GGGAGGACGAUGCGGUCCUGUCGUCUGUCGUCCCCAGACGACUCGCCCGA | 50 |
| Apt19 | 19 | GGGAGGACGAUGCGGUCCUGUCGUCUGUUGUCCCCAGACGACUCGCCCGA | 50 |
| Apt20 | 20 | GGGAGGACGAUGCGGUCCUGUCGUCUGUUCUCCCCAGACGACUCGCCCGA | 50 |
| Apt21 | 21 | GGGAGGACGAUGCGGUCCUGUCGUCUGUUCGCCCCAGACGACUCGCCCGA | 50 |
| Apt22 | 22 | CGGACGAUGCGGUCCGUGCGUCUGUUCGUCCCCAGACGCUCG | 42 |
| Apt23 | 23 | CGGACGAUGCGGUCCGUGUCGCUGUUCGUCCCCAGCGACUCG | 42 |
| Apt24 | 24 | CGGACGAUGCGGUCCGUGUCGUCGUUCGUCCCCGACGACUCG | 42 |
| Apt25 | 25 | CGGACGAUGCGGUCCGUGCGCUGUUCGUCCCCAGCGCUCG | 40 |
| Apt26 | 26 | CGGACGAUGCGGUCCGUGCGUCGUUCGUCCCCGACGCUCG | 40 |
| Apt27 | 27 | CGGACGAUGCGGUCCGUGUCGCGUUCGUCCCCGCGACUCG | 40 |
| Apt28 | 28 | CGGACGAUGCGGUCCGUGCGCGUUCGUCCCCGCGCUCG | 38 |
| Apt29 | 29 | CGGACGAUGCGGUCCGUGUCGCGUUCGUCCCCGCGAC | 37 |
| Apt30 | 30 | GGGAGGACGACGGUCCUGUCGUCUGUUCGUCCCCAGACGACUCGCCCGA | 49 |
| Apt31 | 31 | GGGAGGACGUCGGUCCUGUCGUCUGUUCGUCCCCAGACGACUCGCCCGA | 49 |
| Apt32 | 32 | GGGAGGACAUCGGUCCUGUCGUCUGUUCGUCCCCAGACGACUCGCCCGA | 49 |
| Apt33 | 33 | GGGAGGACGAUGGUCCUGUCGUCUGUUCGUCCCCAGACGACUCGCCCGA | 49 |
| Apt34 | 34 | GGGAGGACGCGGUCCUGUCGUCUGUUCGUCCCCAGACGACUCGCCCGA | 48 |
| Apt35 | 35 | GGGAGGACACGGUCCUGUCGUCUGUUCGUCCCCAGACGACUCGCCCGA | 48 |
| Apt36 | 36 | GGGAGGACGAGGUCCUGUCGUCUGUUCGUCCCCAGACGACUCGCCCGA | 48 |
| Apt37 | 37 | GGGAGGACGGGUCCUGUCGUCUGUUCGUCCCCAGACGACUCGCCCGA | 47 |
| Apt38 | 38 | GGGAGGACAGGUCCUGUCGUCUGUUCGUCCCCAGACGACUCGCCCGA | 47 |
| Apt39 | 39 | GGGAGGACGGUCCUGUCGUCUGUUCGUCCCCAGACGACUCGCCCGA | 46 |
| Apt40 | 40 | GGGAGGACGAUGCGGUCCUGCUCUGUUCGUCCCCAGAGCUCGCCCGA | 47 |
| Apt41 | 41 | GGGAGGACGAUGCGGUCCUGUUCUGUUCGUCCCCAGAACUCGCCCGA | 47 |
| Apt42 | 42 | GGGAGGACGAUGCGGUCCUGCCUGUUCGUCCCCAGGCUCGCCCGA | 45 |
| Apt43 | 43 | GGGAGGACGACGGUCCUGUCCUGUUCGUCCCCAGGACUCGCCCGA | 45 |
| Apt44 | 44 | GGGAGGACGAUCGGUCCUGCUCUGUUCGUCCCCAGAGCUCGCCCGA | 46 |
| Apt45 | 45 | GGGAGGACGACGGUCCUGCUCUGUUCGUCCCCAGAGCUCGCCCGA | 45 |
| Apt46 | 46 | GGGAGGACGAUCGGUCCUGUUCUGUUCGUCCCCAGAACUCGCCCGA | 46 |
| Apt47 | 47 | GGGAGGACGACGGUCCUGUUCUGUUCGUCCCCAGAACUCGCCCGA | 45 |
| Apt48 | 48 | GGGAGGACGACGGUCCUGCCUGUUCGUCCCCAGGCUCGCCCGA | 43 |
| Apt49 | 49 | GGGAGGACGAUCGGUCCUGUCUGUUCGUCCCCAGACUCGCCCGA | 44 |
| Apt50 | 50 | GGGAGGACGACGGUCCUGUCUGUUCGUCCCCAGACUCGCCCGA | 43 |
| Apt51 | 51 | GGGAGGACGCGGUCCUGUCCUGUUCGUCCCCAGGACUCGCCCGA | 44 |
| Apt52 | 52 | GGGAGGACCGGUCCUGUCCUGUUCGUCCCCAGGACUCGCCCGA | 43 |
| Apt53 | 53 | GGGAGGACGGUCCUGUCCUGUUCGUCCCCAGGACUCGCCCGA | 42 |
| Apt54 | 54 | GGAGGACGAUCGGUCCUGUCCUGUUCGUCCCCAGGACUCGCCGA | 44 |
| Apt55 | 55 | GAGGACGAUCGGUCCUGUCCUGUUCGUCCCCAGGACUCGCGA | 42 |
| Apt56 | 56 | GGGUCCUGUCCUGUUCGUCCCCAGGACUCGCCCGA | 35 |
| Apt57 | 57 | GGGUGUCCUGUUCGUCCCCAGGACUCGCCCGA | 32 |
| Apt58 | 58 | GGGGUCCUGUUCGUCCCCAGGACUCGCCCGA | 31 |
| Apt59 | 59 | GGGAGGCGAUCGGCCUGUCCUGUUCGUCCCCAGGACUCGCCCGA | 44 |
| Apt60 | 60 | GGGAGACGAUCGGUCUGUCCUGUUCGUCCCCAGGACUCGCCCGA | 44 |
| Apt61 | 61 | GGGAGCGAUCGGCUGUCCUGUUCGUCCCCAGGACUCGCCCGA | 42 |
| Apt62 | 62 | GGGAGGACCGGUCCUGUCGUCUGUUCGUCCCCAGACGACUCGCCCGA | 47 |
| Apt63 | 63 | GGGAGGACGAUCGGUCCUGUCCUGUUCGUCCCCAGGACUCGCCCGA | 46 |
| Apt64 | 64 | GGGAGGACGAUCGGUCCUGCCUGUUCGUCCCCAGGCUCGCCCGA | 44 |
| Apt65 | 65 | GGGAGGACGGUCCUGUUCUGUUCGUCCCCAGAACUCGCCCGA | 42 |
| Apt66 | 66 | GGGAGGACGGUCCUGUCUGUUCGUCCCCAGACUCGCCCGA | 40 |
| Apt67 | 67 | GAGGACGGUCCUGUCGUCUGUUCGUCCCCAGACGACUCGCGA | 42 |
| Apt68 | 68 | GGGAGCGGCUGUCGUCUGUUCGUCCCCAGACGACUCGCCCGA | 42 |
| Apt69 | 69 | GGAGCGAUGCGGCUGUUCUGUUCGUCCCCAGAACUCGCCGA | 41 |
| Apt70 | 70 | GAGCGAUGCGGCUGUUCUGUUCGUCCCCAGAACUCGCGA | 39 |
| Apt71 | 71 | GAGGACGAUGCGGUCCUGUCUGUUCGUCCCCAGACUCGCGA | 41 |
| Apt72 | 72 | GGGAGCGAUGCGGCUGUCUGUUCGUCCCCAGACUCGCCCGA | 41 |
| Apt73 | 73 | GGAGCGAUGCGGCUGUCUGUUCGUCCCCAGACUCGCCGA | 39 |
| Apt74 | 74 | GAGCGAUGCGGCUGUCUGUUCGUCCCCAGACUCGCGA | 37 |
| Apt75 | 75 | GGAGGACGAUCGGUCCUGUCUGUUCGUCCCCAGACUCGCCGA | 42 |
| Apt76 | 76 | GAGGACGAUCGGUCCUGUUCUGUUCGUCCCCAGAACUCGCGA | 42 |
| Apt77 | 77 | GAGGACGAUCGGUCCUGUCUGUUCGUCCCCAGACUCGCGA | 40 |
| Apt78 | 78 | GGGAGCGAUCGGCUGUUCUGUUCGUCCCCAGAACUCGCCCGA | 42 |
| Apt79 | 79 | GGGAGCGAUCGGCUGUCUGUUCGUCCCCAGACUCGCCCGA | 40 |
| Apt80 | 80 | GGGAGCGACGGCUGUUCUGUUCGUCCCCAGAACUCGCCCGA | 41 |
| Apt81 | 81 | GGAGGACGGUCCUGUCUGUUCGUCCCCAGACUCGCCGA | 38 |
| Apt82 | 82 | GAGGACGGUCCUGUUCUGUUCGUCCCCAGAACUCGCGA | 38 |
| Apt83 | 83 | GAGGACGGUCCUGUCUGUUCGUCCCCAGACUCGCGA | 36 |
| Apt84 | 84 | GGGAGCGGCUGUUCUGUUCGUCCCCAGAACUCGCCCGA | 38 |
| Apt85 | 85 | GGGAGCGGCUGUCUGUUCGUCCCCAGACUCGCCCGA | 36 |
| Apt86 | 86 | GGAGCGAUCGGCUGUUCUGUUCGUCCCCAGAACUCGCCGA | 40 |
| Apt87 | 87 | GGAGCGAUCGGCUGUCUGUUCGUCCCCAGACUCGCCGA | 38 |
| Apt88 | 88 | GAGCGAUCGGCUGUUCUGUUCGUCCCCAGAACUCGCGA | 38 |
| Apt89 | 89 | GAGCGAUCGGCUGUCUGUUCGUCCCCAGACUCGCGA | 36 |
| Apt90 | 90 | GGAGCGACGGCUGUUCUGUUCGUCCCCAGAACUCGCCGA | 39 |
| Apt91 | 91 | GAGCGACGGCUGUUCUGUUCGUCCCCAGAACUCGCGA | 37 |
| Apt92 | 92 | GGAGCGGCUGUUCUGUUCGUCCCCAGAACUCGCCGA | 36 |
| Apt93 | 93 | GGAGCGGCUGUCUGUUCGUCCCCAGACUCGCCGA | 34 |
| Apt94 | 94 | GAGCGGCUGUUCUGUUCGUCCCCAGAACUCGCGA | 34 |
| Apt95 | 95 | GAGCGGCUGUCUGUUCGUCCCCAGACUCGCGA | 32 |
| Apt96 | 96 | GAGCGACGGCUGUCGUCUGUUCGUCCCCAGACGACUCGCGA | 41 |
| Apt97 | 97 | GGAGCGGCUGUCGUCUGUUCGUCCCCAGACGACUCGCCGA | 40 |
| Apt98 | 98 | GAGCGGCUGUCGUCUGUUCGUCCCCAGACGACUCGCGA | 38 |
| Apt99 | 99 | GGAGGACGAGCGGUCCUGUCUGUUCGUCCCCAGACUCGCCGA | 42 |
| Apt100 | 100 | GAGGACGAGCGGUCCUGUUCUGUUCGUCCCCAGAACUCGCGA | 42 |
| Apt101 | 101 | GAGGACGAGCGGUCCUGUCUGUUCGUCCCCAGACUCGCGA | 40 |
| Apt102 | 102 | GGGAGCGAGCGGCUGUUCUGUUCGUCCCCAGAACUCGCCCGA | 42 |
| Apt103 | 103 | GGGAGCGAGCGGCUGUCUGUUCGUCCCCAGACUCGCCCGA | 40 |
| Apt104 | 104 | GGAGCGAGCGGCUGUUCUGUUCGUCCCCAGAACUCGCCGA | 40 |
| Apt105 | 105 | GGAGCGAGCGGCUGUCUGUUCGUCCCCAGACUCGCCGA | 38 |
| Apt106 | 106 | GAGCGAGCGGCUGUUCUGUUCGUCCCCAGAACUCGCGA | 38 |
| Apt107 | 107 | GAGCGAGCGGCUGUCUGUUCGUCCCCAGACUCGCGA | 36 |
| Apt108 | 108 | GAGCGAGCGGCUGUCGUCUGUUCGUCCCCAGACGACUCGCGA | 42 |
| | | | |
| AptP | 109 | GGGAGGACGAUGCGGUCCUUGUCGUCUUGCGUCCCCAGACGACUCGCC CGA | 51 |
| Scramble | 111 | GCACUCGCUCUCGGGCUUGGGCUCUGCCUGCACGGUGGACAACGGCUA GCA | 51 |
| Apt49_UG | 112 | GGGAGGACGAUCGGUCCUGUCUGUGCGUCCCCAGACUCGCCCGA | 44 |
| Apt49_GU | 113 | GGGAGGACGAUCGGUCCUUUCUGUUCGUCCCCAGACUCGCCCGA | 44 |

### Folding prediction

Aptamer folding predictions were carried out using Visual OMP v.7.9.81.0 along with OMP Engine v.4.0.3.0, both from DNA Software (John Santalucia Jr, 2007). The folding temperature was fixed at 37°C with while the salt conditions were set at 150 mM monovalent salt and 10 mM divalent salt (e.g., Mg²⁺). The salt conditions are considered typical physiological conditions. The RNA aptamer concentration was set at 1 nM, although the substrate concentration is relevant only for intermolecular binding. The software was set to return all suboptimal folds within 5 kcal/mole of the optimal folding free energy.

The OMP software uses conventional nearest-neighbour binding free energy calculations to compute the equilibrium folding free energies. The software has free energy parameters for RNA, DNA, and a variety of modified nucleotides, including DNA phosphorothioate, 2'-O-Methyl, LNA, PMO, PNA, and others. In this case, the aptamer was modeled as a pure RNA sequence since the folding software lacks nearest neighbour energy parameters for 2'-fluoro modifications and it has been estimated that the RNA parameters are the closest substitute.

### Example 2.- SPR binding and cell internalization in EphA2 expressing Ewing's sarcoma A673 cell line assays.

The obtained SPR binding and A673 cell internalization results for aptamers Apt1 to Apt108 are shown in Figures 2A to 2C. These figures include AptP for comparison purposes, which was used as reference aptamer.

The aptamers having binding and/or internalization properties superior to those of AptP were considered positive and those with a value lower than AptP negative.

The tables below provide the classification of the aptamers according to their activity:

**Table 2.1. With EphA2 specific binding activity >AptP**

| | | |
|---|---|---|
| Apt11 | GGGAGGACAUGCGGUCCUGUCGUCUGUUCGUCCCCAGACGACUCGCCCGA | 50 |
| Apt12 | GGGAGGACGUGCGGUCCUGUCGUCUGUUCGUCCCCAGACGACUCGCCCGA | 50 |
| Apt14 | GGGAGGACGAUCGGUCCUGUCGUCUGUUCGUCCCCAGACGACUCGCCCGA | 50 |
| Apt15 | GGGAGGACGAUGGGUCCUGUCGUCUGUUCGUCCCCAGACGACUCGCCCGA | 50 |
| Aptl8 | GGGAGGACGAUGCGGUCCUGUCGUCUGUCGUCCCCAGACGACUCGCCCGA | 50 |
| Apt20 | GGGAGGACGAUGCGGUCCUGUCGUCUGUUCUCCCCAGACGACUCGCCCGA | 50 |
| Apt30 | GGGAGGACGACGGUCCUGUCGUCUGUUCGUCCCCAGACGACUCGCCCGA | 49 |
| Apt31 | GGGAGGACGUCGGUCCUGUCGUCUGUUCGUCCCCAGACGACUCGCCCGA | 49 |
| Apt32 | GGGAGGACAUCGGUCCUGUCGUCUGUUCGUCCCCAGACGACUCGCCCGA | 49 |
| Apt33 | GGGAGGACGAUGGUCCUGUCGUCUGUUCGUCCCCAGACGACUCGCCCGA | 49 |
| Apt34 | GGGAGGACGCGGUCCUGUCGUCUGUUCGUCCCCAGACGACUCGCCCGA | 48 |
| Apt35 | GGGAGGACACGGUCCUGUCGUCUGUUCGUCCCCAGACGACUCGCCCGA | 48 |
| Apt36 | GGGAGGACGAGGUCCUGUCGUCUGUUCGUCCCCAGACGACUCGCCCGA | 48 |
| Apt37 | GGGAGGACGGGUCCUGUCGUCUGUUCGUCCCCAGACGACUCGCCCGA | 47 |
| Apt38 | GGGAGGACAGGUCCUGUCGUCUGUUCGUCCCCAGACGACUCGCCCGA | 47 |
| Apt39 | GGGAGGACGGUCCUGUCGUCUGUUCGUCCCCAGACGACUCGCCCGA | 46 |
| Apt40 | GGGAGGACGAUGCGGUCCUGCUCUGUUCGUCCCCAGAGCUCGCCCGA | 47 |
| Apt48 | GGGAGGACGACGGUCCUGCCUGUUCGUCCCCAGGCUCGCCCGA | 43 |
| Apt50 | GGGAGGACGACGGUCCUGUCUGUUCGUCCCCAGACUCGCCCGA | 43 |
| Apt62 | GGGAGGACCGGUCCUGUCGUCUGUUCGUCCCCAGACGACUCGCCCGA | 47 |
| Apt65 | GGGAGGACGGUCCUGUUCUGUUCGUCCCCAGAACUCGCCCGA | 42 |
| Apt72 | GGGAGCGAUGCGGCUGUCUGUUCGUCCCCAGACUCGCCCGA | 41 |
| Apt76 | GAGGACGAUCGGUCCUGUUCUGUUCGUCCCCAGAACUCGCGA | 42 |
| Apt77 | GAGGACGAUCGGUCCUGUCUGUUCGUCCCCAGACUCGCGA | 40 |
| Apt78 | GGGAGCGAUCGGCUGUUCUGUUCGUCCCCAGAACUCGCCCGA | 42 |
| Apt79 | GGGAGCGAUCGGCUGUCUGUUCGUCCCCAGACUCGCCCGA | 40 |
| Apt86 | GGAGCGAUCGGCUGUUCUGUUCGUCCCCAGAACUCGCCGA | 40 |
| Apt87 | GGAGCGAUCGGCUGUCUGUUCGUCCCCAGACUCGCCGA | 38 |

**Table 2.2. With EphA2 binding and cell internalization activity >AptP**

| | | |
|---|---|---|
| Apt1 | GGGAGGACGAUGCGGUCCUGCGUCUGUUCGUCCCCAGACGCUCGCCCGA | 49 |
| Apt2 | GGGAGGACGAUGCGGUCCUGCGCUGUUCGUCCCCAGCGCUCGCCCGA | 47 |
| Apt4 | GGGAGGACGAUGCGGUCCUGUCCUGUUCGUCCCCAGGACUCGCCCGA | 47 |
| Apt6 | GGGAGGACGAUGCGGUCCUGUCUGUUCGUCCCCAGACUCGCCCGA | 45 |
| Apt7 | GGGAGGACGAUGCGGUCCUGUCGUCUGUUCGUCCCCAGACGACUCGCCC | 49 |
| Apt13 | GGGAGGACGAGCGGUCCUGUCGUCUGUUCGUCCCCAGACGACUCGCCCGA | 50 |
| Apt16 | GGGAGGACGAUGCGGUCCUGUCGCUGUUCGUCCCCAGCGACUCGCCCGA | 49 |
| Apt41 | GGGAGGACGAUGCGGUCCUGUUCUGUUCGUCCCCAGAACUCGCCCGA | 47 |
| Apt42 | GGGAGGACGAUGCGGUCCUGCCUGUUCGUCCCCAGGCUCGCCCGA | 45 |
| Apt43 | GGGAGGACGACGGUCCUGUCCUGUUCGUCCCCAGGACUCGCCCGA | 45 |
| Apt44 | GGGAGGACGAUCGGUCCUGCUCUGUUCGUCCCCAGAGCUCGCCCGA | 46 |
| Apt45 | GGGAGGACGACGGUCCUGCUCUGUUCGUCCCCAGAGCUCGCCCGA | 45 |
| Apt46 | GGGAGGACGAUCGGUCCUGUUCUGUUCGUCCCCAGAACUCGCCCGA | 46 |
| Apt47 | GGGAGGACGACGGUCCUGUUCUGUUCGUCCCCAGAACUCGCCCGA | 45 |
| Apt49 | GGGAGGACGAUCGGUCCUGUCUGUUCGUCCCCAGACUCGCCCGA | 44 |
| Apt51 | GGGAGGACGCGGUCCUGUCCUGUUCGUCCCCAGGACUCGCCCGA | 44 |
| Apt52 | GGGAGGACCGGUCCUGUCCUGUUCGUCCCCAGGACUCGCCCGA | 43 |
| Apt53 | GGGAGGACGGUCCUGUCCUGUUCGUCCCCAGGACUCGCCCGA | 42 |
| Apt54 | GGAGGACGAUCGGUCCUGUCCUGUUCGUCCCCAGGACUCGCCGA | 44 |
| Apt55 | GAGGACGAUCGGUCCUGUCCUGUUCGUCCCCAGGACUCGCGA | 42 |
| Apt59 | GGGAGGCGAUCGGCCUGUCCUGUUCGUCCCCAGGACUCGCCCGA | 44 |
| Apt60 | GGGAGACGAUCGGUCUGUCCUGUUCGUCCCCAGGACUCGCCCGA | 44 |
| Apt61 | GGGAGCGAUCGGCUGUCCUGUUCGUCCCCAGGACUCGCCCGA | 42 |
| Apt63 | GGGAGGACGAUCGGUCCUGUCCUGUUCGUCCCCAGGACUCGCCCGA | 46 |
| Apt64 | GGGAGGACGAUCGGUCCUGCCUGUUCGUCCCCAGGCUCGCCCGA | 44 |
| Apt66 | GGGAGGACGGUCCUGUCUGUUCGUCCCCAGACUCGCCCGA | 40 |
| Apt67 | GAGGACGGUCCUGUCGUCUGUUCGUCCCCAGACGACUCGCGA | 42 |
| Apt75 | GGAGGACGAUCGGUCCUGUCUGUUCGUCCCCAGACUCGCCGA | 42 |

**Table 2.3. Negatives (≤ AptP for binding and/or cell internalization properties)**

| | | |
|---|---|---|
| Apt3 | GGGAGGACGAUGCGGUCCUGCGUCGUUCGUCCCCGACGCUCGCCCGA | 47 |
| Apt5 | GGGAGGACGAUGCGGUCCUGCGCGUUCGUCCCCGCGCUCGCCCGA | 45 |
| Apt8 | AGGACGAUGCGGUCCUGUCGUCUGUUCGUCCCCAGACGACUCG | 43 |
| Apt9 | GGACGAUGCGGUCCUGUCGUCUGUUCGUCCCCAGACGACUCG | 42 |
| Apt10 | CGGACGAUGCGGUCCGUGUCGUCUGUUCGUCCCCAGACGACUCG | 44 |
| Apt17 | GGGAGGACGAUGCGUCCUGUCGUCUGUUCGUCCCCAGACGACUCGCCCGA | 50 |
| Apt19 | GGGAGGACGAUGCGGUCCUGUCGUCUGUUGUCCCCAGACGACUCGCCCGA | 50 |
| Apt21 | GGGAGGACGAUGCGGUCCUGUCGUCUGUUCGCCCCAGACGACUCGCCCGA | 50 |
| Apt22 | CGGACGAUGCGGUCCGUGCGUCUGUUCGUCCCCAGACGCUCG | 42 |
| Apt23 | CGGACGAUGCGGUCCGUGUCGCUGUUCGUCCCCAGCGACUCG | 42 |
| Apt24 | CGGACGAUGCGGUCCGUGUCGUCGUUCGUCCCCGACGACUCG | 42 |
| Apt25 | CGGACGAUGCGGUCCGUGCGCUGUUCGUCCCCAGCGCUCG | 40 |
| Apt26 | CGGACGAUGCGGUCCGUGCGUCGUUCGUCCCCGACGCUCG | 40 |
| Apt27 | CGGACGAUGCGGUCCGUGUCGCGUUCGUCCCCGCGACUCG | 40 |
| Apt28 | CGGACGAUGCGGUCCGUGCGCGUUCGUCCCCGCGCUCG | 38 |
| Apt29 | CGGACGAUGCGGUCCGUGUCGCGUUCGUCCCCGCGAC | 37 |
| Apt56 | GGGUCCUGUCCUGUUCGUCCCCAGGACUCGCCCGA | 35 |
| Apt57 | GGGUGUCCUGUUCGUCCCCAGGACUCGCCCGA | 32 |
| Apt58 | GGGGUCCUGUUCGUCCCCAGGACUCGCCCGA | 31 |
| Apt68 | GGGAGCGGCUGUCGUCUGUUCGUCCCCAGACGACUCGCCCGA | 42 |
| Apt69 | GGAGCGAUGCGGCUGUUCUGUUCGUCCCCAGAACUCGCCGA | 41 |
| Apt70 | GAGCGAUGCGGCUGUUCUGUUCGUCCCCAGAACUCGCGA | 39 |
| Apt71 | GAGGACGAUGCGGUCCUGUCUGUUCGUCCCCAGACUCGCGA | 41 |
| Apt73 | GGAGCGAUGCGGCUGUCUGUUCGUCCCCAGACUCGCCGA | 39 |
| Apt74 | GAGCGAUGCGGCUGUCUGUUCGUCCCCAGACUCGCGA | 37 |
| Apt80 | GGGAGCGACGGCUGUUCUGUUCGUCCCCAGAACUCGCCCGA | 41 |
| Apt81 | GGAGGACGGUCCUGUCUGUUCGUCCCCAGACUCGCCGA | 38 |
| Apt82 | GAGGACGGUCCUGUUCUGUUCGUCCCCAGAACUCGCGA | 38 |
| Apt83 | GAGGACGGUCCUGUCUGUUCGUCCCCAGACUCGCGA | 36 |
| Apt84 | GGGAGCGGCUGUUCUGUUCGUCCCCAGAACUCGCCCGA | 38 |
| Apt85 | GGGAGCGGCUGUCUGUUCGUCCCCAGACUCGCCCGA | 36 |
| Apt88 | GAGCGAUCGGCUGUUCUGUUCGUCCCCAGAACUCGCGA | 38 |
| Apt89 | GAGCGAUCGGCUGUCUGUUCGUCCCCAGACUCGCGA | 36 |
| Apt90 | GGAGCGACGGCUGUUCUGUUCGUCCCCAGAACUCGCCGA | 39 |
| Apt91 | GAGCGACGGCUGUUCUGUUCGUCCCCAGAACUCGCGA | 37 |
| Apt92 | GGAGCGGCUGUUCUGUUCGUCCCCAGAACUCGCCGA | 36 |
| Apt93 | GGAGCGGCUGUCUGUUCGUCCCCAGACUCGCCGA | 34 |
| Apt94 | GAGCGGCUGUUCUGUUCGUCCCCAGAACUCGCGA | 34 |
| Apt95 | GAGCGGCUGUCUGUUCGUCCCCAGACUCGCGA | 32 |
| Apt96 | GAGCGACGGCUGUCGUCUGUUCGUCCCCAGACGACUCGCGA | 41 |
| Apt97 | GGAGCGGCUGUCGUCUGUUCGUCCCCAGACGACUCGCCGA | 40 |
| Apt98 | GAGCGGCUGUCGUCUGUUCGUCCCCAGACGACUCGCGA | 38 |
| Apt99 | GGAGGACGAGCGGUCCUGUCUGUUCGUCCCCAGACUCGCCGA | 42 |
| Apt100 | GAGGACGAGCGGUCCUGUUCUGUUCGUCCCCAGAACUCGCGA | 42 |
| Apt101 | GAGGACGAGCGGUCCUGUCUGUUCGUCCCCAGACUCGCGA | 40 |
| Apt102 | GGGAGCGAGCGGCUGUUCUGUUCGUCCCCAGAACUCGCCCGA | 42 |
| Apt103 | GGGAGCGAGCGGCUGUCUGUUCGUCCCCAGACUCGCCCGA | 40 |
| Apt104 | GGAGCGAGCGGCUGUUCUGUUCGUCCCCAGAACUCGCCGA | 40 |
| Apt105 | GGAGCGAGCGGCUGUCUGUUCGUCCCCAGACUCGCCGA | 38 |
| Apt106 | GAGCGAGCGGCUGUUCUGUUCGUCCCCAGAACUCGCGA | 38 |
| Apt107 | GAGCGAGCGGCUGUCUGUUCGUCCCCAGACUCGCGA | 36 |
| Apt108 | GAGCGAGCGGCUGUCGUCUGUUCGUCCCCAGACGACUCGCGA | 42 |

To sum up, we can conclude that Apt4, 6, 46, 47, 49, 54, 61, 63, 64, 66 and 67 are the best candidate aptamers, showing better results in both SPR binding and *in vitro* internalization assays. Indeed, the group of best candidate aptamers were those characterized by having cell internalization values of at least 5-fold over AptP.

### Example 3.- Functional assays

### 3.A. Migration assay

Migration assays were performed with some of the best performing candidates (Apt46, Apt47, Apt49, Apt63, Apt66 and Apt75), as described in Materials and Methods. Cells were seeded in on the top of the transwell. After 24h, migrating purple stained cells (shown in dark grey) which were able to go through the transwell were quantified. Any aptamer treated cells resulted in a significant reduction of the migratory and invasive capacity of A673 cells *in vitro*, compared to the untreated cells (Empty) **(****Fig.3A****).** From the tested candidates, the best ones were found to be Apt46 and Apt49, followed by Apt66 **(****Fig.3B****).**

The observed significant reduction of the migratory and invasive capacity of the EphA2 expressing cells suggests the anti-cancer and more specifically anti-metastatic properties of the tested aptamers on their own, with Apt46, Apt49 and Apt66 showing the best results.

### 3.B. Cell internalization assay in other EphA2 cancer types

EphA2 receptor is highly expressed in different types of cancer, while it is mainly not produced in normal adult tissues (Xiao T et al, 2020) **(****Fig.3C****).** EphA2 receptor expression is also linked to poor prognosis and survival (London, M. et al, 2020). Thus, pancreatic, bladder, ovarian, brain, head and neck, uterine, colorectal, kidney, sarcoma, cervical, rhabdomyosarcoma, prostate and breast cancers, among others, are some examples of cancers with EphA2 overexpression.

For this reason, we performed internalization assays with different EphA2 receptor expressing cell lines, MDA-MB231 as a breast cancer and RH4 as a rhabdomysarcoma cell line. As it is shown in **Fig.3D**, Apt49 aptamer strongly internalizes in any of the tested cell lines. The Apt66 aptamer seems to better internalize in A673 cell line.

### Example 4.- Chemical modifications

This assay has as main objective to elucidate modifications that will improve stability without affecting the internalization capacity of the Apt49 aptamer **(****Fig.4A****).** As described under material and methods above, the starting product was Apt49 which is characterized by being 2'F-modified in the pyrimidines.

### 4.A. Phosphorothioate (PTO) modifications

Phosphorothioate (PTO) modifications were added to Apt49 aptamer as previously described by Lennox, KA et al (Lennox, KA et al, 2011) to prevent degradation by nucleases while being in circulation or inside the cell. The modifications were performed in all the nucleotides of the respective blocks: the aptamer loops (L1, L2 and L3) and at the end of the molecule (Ends), respectively, when considering a 3-stem conformation (see Fig. 1A.2). As it is shown in **Fig.4B**, the addition of PTOs at the ends of the aptamer increased its internalization capacity, although not statistically significant.

### 4.B. 2'-O-methylation (2'OMe) modifications

In another assay, Apt49 was modified by alternating 2'F/2'OMe in the selected blocks **(****Fig.4C****).** It is noted that none of the variants showed better internalization than Apt49. However, it was observed that when the alternating 2'F/2'OMe modification was performed one by one, some candidates showed higher internalization capacity. As it is shown in **Fig.4D**, the internalization was higher when the positions 2, 9, 10, 13, 16, 19 from Apt49 were modified by 2'OMes.

Table 3 below shows the sequences of the various chemically modified aptamers assayed. All these are versions of the Apt49 candidate.

**Table 3. - Apt49 versions including (PTO) or 2'OMe modifications**

| **ID** | **SEQ ID Nº** | **Sequence** | **nts** |
|---|---|---|---|
| PTO_Ends | 114 | GsGsGAGGACGAUCGGUCCUGUCUGUUCGUCCCCAGACUCGCCCsGsA | 44 |
| PTO_L1 | 115 | GGGAGGACsGsAsUsCsGsGUCCUGUCUGUUCGUCCCCAGACUCGCCCGA | 44 |
| PTO_L2 | 116 | GGGAGGACGAUCGGUCCUGUCUGsUsUsCsGsUsCsCsCsCAGACUCGCCCGA | 44 |
| PTO_L3 | 117 | GGGAGGACGAUCGGUCCUGUCUGUUCGUCCCCAGACsUsCsGsCCCGA | 44 |
| OMe_L1 | 118 | GGGAGGACgaucgGUCCUGUCUGUUCGUCCCCAGACUCGCCCGA | 44 |
| OMe_L2.1 | 119 | GGGAGGACGAUCGGUCCUGUCUGuucgUCCCCAGACUCGCCCGA | 44 |
| OMe_L2.2 | 120 | GGGAGGACGAUCGGUCCUGUCUGUUCGucccCAGACUCGCCCGA | 44 |
| OMe_L3 | 121 | GGGAGGACGAUCGGUCCUGUCUGUUCGUCCCCAGACucgCCCGA | 44 |
| OMe_S1 | 122 | gggAGGACGAUCGGUCCUGUCUGUUCGUCCCCAGACUCGcccGA | 44 |
| OMe_S2 | 123 | GGGaggacGAUCGguccuGUCUGUUCGUCCCCAGACUCGCCCGA | 44 |
| OMe_S3 | 124 | GGGAGGACGAUCGGUCCUgucugUUCGUCCCcagaCUCGCCCGA | 44 |
| OMe_1 | 125 | GGGAGGAcGAUCGGUCCUGUCUGUUCGUCCCCAGACUCGCCCGA | 44 |
| OMe_2 | 126 | GGGAGGACGAuCGGUCCUGUCUGUUCGUCCCCAGACUCGCCCGA | 44 |
| OMe_3 | 127 | GGGAGGACGAUcGGUCCUGUCUGUUCGUCCCCAGACUCGCCCGA | 44 |
| OMe_4 | 128 | GGGAGGACGAUCGGuCCUGUCUGUUCGUCCCCAGACUCGCCCGA | 44 |
| OMe_5 | 129 | GGGAGGACGAUCGGUcCUGUCUGUUCGUCCCCAGACUCGCCCGA | 44 |
| OMe_6 | 130 | GGGAGGACGAUCGGUCcUGUCUGUUCGUCCCCAGACUCGCCCGA | 44 |
| OMe_7 | 131 | GGGAGGACGAUCGGUCCuGUCUGUUCGUCCCCAGACUCGCCCGA | 44 |
| OMe_8 | 132 | GGGAGGAcGAUCGGUCCUGUCUGUUCGUCCCCAGACUCGCCCGA | 44 |
| OMe_9 | 133 | GGGAGGACGAUCGGUCCUGUcUGUUCGUCCCCAGACUCGCCCGA | 44 |
| OMe_10 | 134 | GGGAGGAcGAUCGGUCCUGUCUGUUCGUCCCCAGACUCGCCCGA | 44 |
| OMe_11 | 135 | GGGAGGACGAUCGGUCCUGUCUGuUCGUCCCCAGACUCGCCCGA | 44 |
| OMe_12 | 136 | GGGAGGACGAUCGGUCCUGUCUGUuCGUCCCCAGACUCGCCCGA | 44 |
| OMe_13 | 137 | GGGAGGACGAUCGGUCCUGUCUGUUcGUCCCCAGACUCGCCCGA | 44 |
| OMe_14 | 138 | GGGAGGACGAUCGGUCCUGUCUGUUCGuCCCCAGACUCGCCCGA | 44 |
| OMe_15 | 139 | GGGAGGACGAUCGGUCCUGUCUGUUCGUcCCCAGACUCGCCCGA | 44 |
| OMe_16 | 140 | GGGAGGACGAUCGGUCCUGUCUGUUCGUCcCCAGACUCGCCCGA | 44 |
| OMe_17 | 141 | GGGAGGAcGAUCGGUCCUGUCUGUUCGUCCCCAGACUCGCCCGA | 44 |
| OMe_18 | 142 | GGGAGGACGAUCGGUCCUGUCUGUUCGUCCCcAGACUCGCCCGA | 44 |
| OMe_19 | 143 | GGGAGGACGAUCGGUCCUGUCUGUUCGUcCCCAGACUCGCCCGA | 44 |
| OMe_20 | 144 | GGGAGGAcGAUCGGUCCUGUCUGUUCGUCCCCAGACUCGCCCGA | 44 |
| OMe_21 | 145 | GGGAGGACGAUCGGUCCUGUCUGUUCGUcCCCAGACUCGCCCGA | 44 |
| OMe_22 | 146 | GGGAGGACGAUCGGUCCUGUCUGUUCGUCCCCAGACUCGcCCGA | 44 |
| OMe_23 | 147 | GGGAGGACGAUCGGUCCUGUCUGUUCGUcCCCAGACUCGCCCGA | 44 |
| OMe_24 | 148 | GGGAGGACGAUCGGUCCUGUCUGUUCGUcCCCAGACUCGCCCGA | 44 |

| **LEGEND** | | | |
|---|---|---|---|
| RNA bases | capital letters | | |
| Phosphorothioate (PTO) | s | | |
| 2'OMe | lowercase bases | | |

### Example 5.- Apt49 variants - Relevance of the nucleotides at positions 20 and 29 of the aptamer of formula I for aptamer functionality

To highlight the importance of the nucleotides at positions 20 and 29 of the aptamer of formula I, a specific comparison assay was designed where the corresponding bases within the Apt49 sequence were mutated. Accordingly, in the original sequence Apt49 n₂₀ is G, whereas n₂₉ is U.

The tested variants of Apt49 were as follows:
i) Apt49_UG: the U at position 29 was exchanged by G;
ii) Apt49_GU: the G at position 20 was exchanged by U.

See the corresponding sequences in Table 4 below:

| | | |
|---|---|---|
| Apt49_UG | GGGAGGACGAUCGGUCCUGUCUGUGCGUCCCCAGACUCGCCCGA | 44 |
| Apt49_GU | GGGAGGACGAUCGGUCCUUUCUGUUCGUCCCCAGACUCGCCCGA | 44 |

**Fig.5A** shows the corresponding 3D-conformation structures using RNAfold WebServer, the change in the nucleotide at position 20 resulted in the loss of the 3-stem structure for Apt49_GU.

Moreover, as illustrated by **Fig.5B** the internalization capability dropped completely for both mutated variants. The obtained results suggest that the specific base at these two positions were playing a role in the aptamer internalization and are thus critical when aptamer internalization is desired.

### Example 6- Predicted motifs in the Folded Structures

For each of the generated aptamers, the folded structures were analyzed for the number of stems present in the most stable structure, as well as the number of nucleotides in each of the stems, loops and linkers between stems. This analysis was performed using the OMP DE from (DNA Software, USA). For a given aptamer sequence, the program based on proprietary integrative nucleic acids folding algorithms predicts what is the more likely 3D-structure of the aptamer and creates a ranking indicating in what percentage a given structure is predicted to be present.

It was found that from the 56 positive sequences, 44 were predicted to form a 3-stem structure as their most stable fold, and in all these cases the 3-stem structure was predicted to represent ≥80%. See for illustrative purposes Fig. 1B and 1C. Moreover, Fig. 6 shows the 3-stem structure of Apt49 which is one of the more preferred aptamers of the invention.

We further looked into the suboptimal folds for the 4 positive outliers (Apt34, Apt75, Apt67 and Apt68) and found that the 3-stem structure is predicted as one of the suboptimal folds in each case. For Apt75, the 3-stem structure is predicted to represent 35.8% of the folded structures.

In conclusion, the ability of the aptamer to form a 3-stem structure has positively been associated with the presence of activity.

### Example 7- SPR Kinetic Measurements

SPR kinetic measurements were obtained for exemplary binding curves of the Apt49, Apt54 and Apt60 candidates.

For the on-off (association-dissociation) rate map the calculated kₐ mean values were plotted against the k_{d} mean values. The exemplary diagonals represent the K_{d} values of 0.1 nM and 1 nM.

| | k_{d} [s-¹] | kₐ [M⁻¹s⁻¹] |
|---|---|---|
| Apt49 | 0,77e-3 | 4,76e6 |
| Apt54 | 0,68e-3 | 3,75e6 |
| Apt60 | 1,41e-3 | 2,76e6 |

Thus, the on-off rate map visualizes the relationship between kinetics (kₐ, k_{d}) and affinity K_{d} (K_{d} = k_{d}/kₐ). Aptamers with the same affinity K_{d} (points on the same diagonal) can result from different kinetics of these aptamers.

### References

Xiao T, Xiao Y, Wang W, Tang YY, Xiao Z, Su M. Targeting EphA2 in cancer. J Hematol Oncol. 2020 Aug 18;13(1):114. doi: 10.1186/s13045-020-00944-9. PMID: 32811512; PMCID: PMC7433191.
Tandon et al.. Emerging strategies for EphA2 receptor targeting for cancer therapeutics. Expert Opin Ther Targets. 2011; 15(1): 31-51.
Kasinski and Slack, Small RNAs deliver a blow to ovarian cancer. Cancer Discov. 2013; 3: 1220-1221.
Quinn et al.. Therapy of pancreatic cancer via an EphA2 receptor-targeted delivery of Gemcitabine. Oncotarget. 2016; 7: 17103-17110.
London M, Gallo E. The EphA2 and cancer connection: potential for immune-based interventions. Mol Biol Rep. 2020 Oct;47(10):8037-8048. doi: 10.1007/s11033-020-05767-y. Epub 2020 Sep 29. PMID: 32990903.
Lennox KA, Behlke MA. Chemical modification and design of anti-miRNA oligonucleotides. Gene Ther. 2011 Dec;18(12):1111-20. doi: 10.1038/gt.2011.100. Epub 2011 Jul 14. PMID: 21753793.
Thaker, P.H., Deavers, M., Celestino, J., Thornton, A., Fletcher, M.S., Landen, C.N., Kinch,M.S., Kiener, P.A., and Sood, A.K. (2004). EphA2 expression is associated with aggressive features in ovarian carcinoma. Clin. Cancer Res. 10, 5145-5150.
Lin, Y.G., Han, L.Y., Kamat, A.A., Merritt, W.M., Landen, C.N., Deavers, M.T., Fletcher, M.S., Urbauer, D.L., Kinch, M.S., and Sood, A.K. (2007). EphA2 overexpression is associated with angiogenesis in ovarian cancer. Cancer 109, 332-340.
Walker-Daniels, J., Coffman, K., Azimi, M., Rhim, J.S., Bostwick, D.G., Snyder, P., Kerns, B.J., Waters, D.J., and Kinch, M.S. (1999). Overexpression of the EphA2 tyrosine kinase in prostate cancer. Prostate 41, 275-280.
Zhao, Y., Cai, C., Zhang, M., Shi, L., Wang, J., Zhang, H., Ma, P., and Li, S. (2021). Ephrin-A2 promotes prostate cancer metastasis by enhancing angiogenesis and promoting EMT. J. Cancer Res. Clin. Oncol. 147, 2013-2023.
Duxbury, M.S., Ito, H., Zinner, M.J., Ashley, S.W., and Whang, E.E. (2004). EphA2: a determinant of malignant cellular behavior and a potential therapeutic target in pancreatic adenocarcinoma. Oncogene 23, 1448-1456.
Wykosky, J., Gibo, D.M., Stanton, C., and Debinski, W. (2005). EphA2 as a novel molecular marker and target in glioblastoma multiforme. Mol. Cancer Res. 3, 541-551.
Wang, L.F., Fokas, E., Bieker, M., Rose, F., Rexin, P., Zhu, Y., Pagenstecher, A., Engenhart-Cabillic, R., and An, H.X. (2008). Increased expression of EphA2 correlates with adverse outcome in primary and recurrent glioblastoma multiforme patients. Oncol. Rep. 19, 151-156.
Kinch, M.S., Moore, M.-B., and Harpole, D.H. (2003). Predictive value of the EphA2 receptor tyrosine kinase in lung cancer recurrence and survival. Clin. Cancer Res. 9, 613-618.
Margaryan, N.v., Strizzi, L., Abbott, D.E., Seftor, E.A., Rao, M.S., Hendrix, M.J.C., and Hess, A.R. (2009). EphA2 as a promoter of melanoma tumorigenicity. Cancer Biol. Ther. 8, 279-288.
Udayakumar, D., Zhang, G., Ji, Z., Njauw, C.N., Mroz, P., and Tsao, H. (2011). Epha2 is a critical oncogene in melanoma. Oncogene 30, 4921-4929.
Miyazaki, T., Kato, H., Fukuchi, M., Nakajima, M., and Kuwano, H. (2003). EphA2 overexpression correlates with poor prognosis in esophageal squamous cell carcinoma. Int. J. Cancer 103, 657-663.
Dunne, P.D., Dasgupta, S., Blayney, J.K., McArt, D.G., Redmond, K.L., Weir, J.A., Bradley, C.A., Sasazuki, T., Shirasawa, S., Wang, T., et al. (2016). EphA2 expression is a key driver of migration and invasion and a poor prognostic marker in colorectal cancer. Clin. Cancer Res. 22, 230-242.
Kataoka, H., Igarashi, H., Kanamori, M., Ihara, M., Wang, J.D., Wang, Y.J., Li, Z.Y., Shimamura, T., Kobayashi, T., Maruyama, K., et al. (2004). Correlation of EPHA2 overexpression with high microvessel count in human primary colorectal cancer. Cancer Sci. 95, 136-141.
Saito, T., Masuda, N., Miyazaki, T., Kanoh, K., Suzuki, H., Shimura, T., Asao, T., and Kuwano, H. (2004). Expression of EphA2 and E-cadherin in colorectal cancer: correlation with cancer metastasis. Oncol. Rep. 11, 605-611.
Giordano, G., Merlini, A., Ferrero, G., Mesiano, G., Fiorino, E., Brusco, S., Centomo, M.L., Leuci, V., D'ambrosio, L., Aglietta, M., et al. (2021). EphA2 expression in bone sarcomas: bioinformatic analyses and preclinical characterization in patient-derived models of osteosarcoma, ewing's sarcoma and chondrosarcoma. Cells 10, 2893.
Posthumadeboer, J., Piersma, S.R., Pham, T.v., van Egmond, P.W., Knol, J.C., Cleton-Jansen, A.M., van Geer, M.A., van Beusechem, V.W., Kaspers, G.J.L., van Royen, B.J., et al. (2013). Surface proteomic analysis of osteosarcoma identifies EPHA2 as receptor for targeted drug delivery. Br. J. Cancer 109, 2142-2154.
Zhang, X. (2021). The expression profile and prognostic values of EPHA family members in breast cancer. Front. Oncol. 11, 619949.
Brantley-Sieders, D.M., Zhuang, G., Hicks, D., Fang, W.B., Hwang, Y., Cates, J.M.M., Coffman, K., Jackson, D., Bruckheimer, E., Muraoka-Cook, R.S., and Chen, J. (2008). The receptor tyrosine kinase EphA2 promotes mammary adenocarcinoma tumorigenesis and metastatic progression in mice by amplifying ErbB2 signaling. J. Clin. Invest. 118, 64-78.
Zelinski, D.P., Zantek, N.D., Stewart, J.C., Irizarry, A.R., and Kinch, M.S. (2001). EphA2 overexpression causes tumorigenesis of mammary epithelial cells. Cancer Res. 61, 2301-2306.
Brantley-Sieders, D.M., Fang, W.B., Hicks, D.J., Zhuang, G., Shyr, Y., and Chen, J. (2005). Impaired tumor microenvironment in EphA2-deficient mice inhibits tumor angiogenesis and metastatic progression. Faseb. J. 19, 1884-1886.
Carles-Kinch, K., Kilpatrick, K.E., Stewart, J.C., and Kinch, M.S. (2002). Antibody targeting of the EphA2 tyrosine kinase inhibits malignant cell behavior. Cancer Res. 62, 2840-2847.
Yang, Y., Nian, S., Li, L., Wen, X., Liu, Q., Zhang, B., Lan, Y., Yuan, Q., and Ye, Y. (2021). Fully human recombinant antibodies against EphA2 from a multi-tumor patient immune library suitable for tumor-targeted therapy. Bioengineered 12, 10379-10400.
Biao-xue, R., Xi-guang, C., Shuan-ying, Y., Wei, L., and Zong-juan, M. (2011). EphA2-Dependent molecular targeting therapy for malignant tumors. Curr. Cancer Drug Targets 11, 1082-1097.
Jackson, D., Gooya, J., Mao, S., Kinneer, K., Xu, L., Camara, M., Fazenbaker, C., Fleming, R., Swamynathan, S., Meyer, D., et al. (2008). A human antibody-drug conjugate targeting EphA2 inhibits tumor growth in vivo. Cancer Res. 68, 9367-9374.
Koolpe, M., Dail, M., and Pasquale, E.B. (2002). An ephrin mimetic peptide that selectively targets the EphA2 receptor. J. Biol. Chem. 277, 46974-46979.
Wang, S., Placzek, W.J., Stebbins, J.L., Mitra, S., Noberini, R., Koolpe, M., Zhang, Z., Dahl, R., Pasquale, E.B., and Pellecchia, M. (2012). Novel targeted system to deliver chemotherapeutic drugs to EphA2-expressing cancer cells. J. Med. Chem. 55, 2427-2436.
Odeh, F., Nsairat, H., Alshaer, W., Ismail, M.A., Esawi, E., Qaqish, B., Bawab, A.A., Ismail, S.I., and Jo, A.A.B. (2019). Aptamers chemistry: chemical modifications and conjugation strategies. Molecules 25, 3.
Adachi, T., & Nakamura, Y. (2019). Aptamers: A Review of Their Chemical Properties and Modifications for Therapeutic Application. Molecules (Basel, Switzerland), 24(23), 4229.Beaucage, S. L.; Lyer, R. P. (1992). "Advances in the Synthesis of Oligonucleotides by the Phosphoramidite Approach". Tetrahedron. 48 (12): 2223.
Chen Z, Luo H, Gubu A, Yu S, Zhang H, Dai H, Zhang Y, Zhang B, Ma Y, Lu A, Zhang G. (2023). Chemically modified aptamers for improving binding affinity to the target proteins via enhanced non-covalent bonding. Front Cell Dev Biol. 11:1091809.
Santana-Viera L, Dassie JP, Rosàs-Lapeña M, Garcia-Monclús S, Chicón-Bosch M, Pérez-Capó M, Pozo LD, Sanchez-Serra S, Almacellas-Rabaiget O, Maqueda-Marcos S, López-Alemany R, Thiel WH, Giangrande PH, Tirado OM. Combination of protein and cell internalization SELEX identifies a potential RNA therapeutic and delivery platform to treat EphA2-expressing tumors. Mol Ther Nucleic Acids. 2023 May 8;32:758-772.
John Santalucia, Jr. (2007) "Physical principles and visual-OMP software for optimal PCR design." in Methods in Molecular Biology, Humana Press, 402:3-34, 1-59745-528-8:3
Zachary J Kartje, Helen I Janis, Shaoni Mukhopadhyay, Keith T Gagnon Revisiting T7 RNA polymerase transcription in vitro with the Broccoli RNA aptamer as a simplified real-time fluorescent reporter J Biol Chem. 2021 Jan-Jun;296:100175
Kristina W. Thiel, Luiza I. Hernandez, Justin P. Dassie, William H. Thiel, Xiuying Liu, Katie R. Stockdale, Alissa M. Rothman, Frank J. Hernandez, James O. McNamara and Paloma H. Giangrande Delivery of chemo-sensitizing siRNAs to HER2+-breast cancer cells using RNA aptamers Nucleic Acids Research, 2012, Vol. 40, No. 13 6319-6337
Paula E Burmeister, Scott D Lewis, Robert F Silva, Jeffrey R Preiss, Lillian R Horwitz, P Shannon Pendergrast, Thomas G McCauley, Jeffrey C Kurz, David M Epstein, Charles Wilson, Anthony D Keefe Direct in vitro selection of a 2'-O-methyl aptamer to VEGF Chem Biol. 2005 Jan;12(1):25-33
Michael Kohlberger, Gabriele Gadermaier SELEX: Critical factors and optimization strategies for successful aptamer selection Biotechnol Appl Biochem. 2022;69:1771-1792.
A Jäschke, J P Fürste, E Nordhoff, F Hillenkamp, D Cech, and V A Erdmann Synthesis and properties of oligodeoxyribonucleotide-polyethylene glycol conjugates Nucleic Acids Res. 1994 Nov 11; 22(22): 4810-4817.
Kölmel, Dominik K.; Kool, Eric T. (2017). "Oximes and Hydrazones in Bioconjugation: Mechanism and Catalysis". Chemical Reviews. 117 (15): 10358-10376.
Doronina, S. O. et al. Development of potent monoclonal antibody auristatin conjugates for cancer therapy. Nat. Biotechnol. 21, 778-784 (2003)

### ITEMS OF THE INVENTION

1. A nucleic acid aptamer which binds specifically to EphA2, preferably human EphA2, the aptamer comprising the three polynucleotide regions Region 1, Region 2 and Region 3 defined below provided in the 5' to 3' order of the aptamer, wherein:
   i. Region 1 is of formula (R1):

      5' n₁ n₂ G A G n₆ n₇ C n₉ n₁₀ n₁₁ n₁₂ n₁₃ G G n₁₆ n₁₇ C 3'

      wherein:
      n₁ and n₂ are independently selected from G, dG or - ;
      n₉ is G, dG or-;
      n₇ is A, dA or-;
      n₉ is G, dG or-;
      n₁₀ is A, dA or-;
      n₁₁ is U, dU or-;
      n₁₂ is G, dG or-;
      n₁₃ is C, dC or-;
      ₀₁₆ is U, dU or-;
      n₁₇ is C, dC or -;
      wherein G, A, U and C are ribonucleotides; dG, dA, dU and dC are 2'-deoxyribonucleotides wherein the respective ribonucleotide or
      deoxyribonucleotide includes modified forms thereof, and "-" means absence of nucleotide;
      provided that
      n₉ and n₁₇ are complementary nucleotides or, alternatively, are absent;
      n₇ and n₁₆ are complementary nucleotides or, alternately, are absent;
      when n₁ is -, n₁₂ is -; and
      when n₇ is -, n₁₁ is U or dU;
   ii. Region 2 is of formula (R2):

      5' U G n₂₁ n₂₂ n₂₃ n₂₄ n₂₅ U G n₂₈ U C n₃₁ U C C C C A n₃₈ n₃₉ n₄₀ n₄₁ n₄₂ 3'

      wherein:
      n₂₁ is U, dU or
      n₂₂ is C, dC or-;
      n₂₃ is G, dG or-;
      n₂₄ is U, dU or-;
      n₂₅ is C, dC or -;
      n₂₈ is U, dU or
      n₃₁ is G, dG or-;
      n₃₈ is G, dG or-;
      n₃₉ is A, dA or-;
      n₄₀ is C, dC or-;
      n₄₁ is G, dG or-;
      n₄₂ is A, dA or-;
      provided that:
      n₂₁ and n₄₂ are complementary nucleotides or, alternatively, are absent;
      n₂₂ and n₄₁ are complementary nucleotides or, alternatively, are absent;
      n₂₃ and n₄₀ are complementary nucleotides or, alternatively, are absent;
      n₂₄ and n₃₉ are complementary nucleotides or, alternatively, are absent; and
      n₂₅ and n₃₈ are complementary nucleotides or, alternatively, are absent;
   iii. Region 3 is of formula (R3):

      5' C U C G C n₄₈ n₄₉ n₅₀ n₅₁ 3'

      wherein:
      n₄₈ is C, dC or -;
      n₄₉ is C, dC or -;
      n₅₀ is G, dG or-;
      n₅₁ is A, dA or -;
      provided that
      n₁ of R1 and n₄₉ of R3 are complementary nucleotides or, alternatively, are absent; and
      n₂ of R1 and n₄₈ of R3 are complementary nucleotides or, alternatively, are absent;
      preferably, wherein said aptamer is further characterized by:
      a) having a length of 50 nucleotides or less; and/or
      b) n₁₁ and/or n₁₂ of R1 is absent.
2. The nucleic acid aptamer of item 1, which comprises or consists of the sequence of formula (I):

   5' n₁ n₂ G A G n₆ n₇ C n₉ n₁₀ n₁₁ n₁₂ n₁₃ G G n₁₆ n₁₇ C U G n₂₁ n₂₂ n₂₃ n₂₄ n₂₅ U G n₂₈ U C n₃₁ U C C C C A n₃₈ n₃₉ n₄₀ n₄₁ n₄₂ C U C G C n₄₈ n₄₉ n₅₀ n₅₁, 3,

   wherein nucleotides n₁, n₂, n₆, n₇, n₉ to n₁₃, n₁₆, n₁₇, n₂₁ to n₂₅, n₂₈, n₃₁, n₃₈ to n₄₂ and n₄₈ to n₅₁ are as defined in item 1, preferably wherein n₂₅ is C and n₂₈ is U.
3. The nucleic acid aptamer according to any one of the preceding items, wherein said nucleotides are ribonucleotides.
4. The nucleic acid aptamer of items 1 to 3, wherein
   i. when two or more of n₉ to n₁₃ are absent, then n₉ and n₁₇ are complementary nucleotides and n₇ and n₁₆ are complementary nucleotides; and/or
   ii. when only one of n₉ to n₁₃ is absent, then n₁₁ or n₁₂ is absent.
5. The nucleic acid aptamer of any one of items 1 to 4, wherein all the nucleotides from n₉ to n₁₃ are absent, n₆ is complementary to n₁₇, and n₇ is complementary to n₁₆.
6. The nucleic acid aptamer of any one of items 1 to 5, wherein n₂₃ and n₄₀ are absent; and
   n₂₁ is complementary to n₄₂; and/or
   n₂₅ is complementary to n₃₈.
7. The nucleic acid aptamer of any one of any one of items 1 to 6, wherein
   n₂₂ and n₄₁ are absent;
   n₂₃ and n₄₀ are absent; and
   n₂₄ and n₃₉ are absent; and
   preferably, n₉ and n₁₇ are complementary nucleotides; and
   preferably, n₇ and n₁₆ are complementary nucleotides.
8. The nucleic acid aptamer of any one of the preceding items, wherein n₂₁ is U, n₂₅ is C, n₂₈ is U, n₃₁ is G, n₃₈ is G and n₄₂ is A.
9. The nucleic acid aptamer of any one of the preceding items, wherein
   n₁ of R1 and n₄₉ of R3 are absent; and/or
   n₂ of R1 and n₄₈ of R3 are absent;
   n₅₀ is G and n₅₁ is A.
10. The nucleic acid aptamer of any one of the preceding items, wherein
   n₁ of R1 and n₄₉ of R3 are complementary;
   n₂ of R1 and n₄₈ of R3 are complementary; and
   n₅₀ is G and n₅₁ is A.
11. A nucleic acid aptamer which binds specifically to EphA2, preferably human EphA2, wherein said nucleic acid aptamer is selected from the group consisting of the sequences identified in Table 2 .1 and/or 2.2, or a variant having at least 85%, preferably at least 90% identity, more preferably at least 95% identity to any thereof.
12. The nucleic acid aptamer of any one of items 1 to 10, wherein the aptamer is selected from the group consisting of the sequences identified in Table 2 .1 and/or 2.2, or a variant having at least 85%, preferably at least 90% identity, more preferably at least 95% identity to any thereof.
13. The nucleic acid aptamer of any one of the preceding items, wherein the aptamer is further characterized by when using a software for folding prediction, such as the OMP DE from DNA Software a 3-stem structure is predicted to represent at least 35% of the folded structures.
14. The nucleic acid aptamer of any one of the preceding items, wherein the aptamer has at least 38 nucleotides, preferably from 40 to 50 nucleotides.
15. The nucleic acid aptamer of any one of the preceding items, wherein the aptamer is characterized by having one or more of the following characteristics:
   a. a binding response superior to AptP as determined by surface plasmon resonance (SPR) using a Biacore biosensor system, preferably wherein the determined value (e.g., at the end of the dissociation) for the aptamer is at least one-fold higher, more preferably at least 1.5 or 2-fold higher than that of AptP;
   b. binds to human EphA2 with a KD of 1 × 10⁻⁹ M or less, as determined by SPR using a Biacore biosensor system;
   c. has internalization properties in EphA2 expressing cells superior to AptP as determined by RT-qPCR cell internalization assay, preferably wherein the determined value for the aptamer is at least one-fold higher, more preferably at least 1.5 or 2-fold higher than that of AptP; and/or
   d. reduces *in vitro* migration of EphA2 expressing cells, such as A673 cells, with respect to untreated cells or a control aptamer.
16. The nucleic acid aptamer of any one of the preceding items, wherein one or more of the nucleotides is chemically modified, preferably wherein said chemical modification includes one or more of:
   i. modifications on the sugar ring, such as 2' -substitutions, 4' -oxygen replacement, locked and unlocked nucleic acids,
   ii. modifications on the phosphodiester linkage, such as methylphosphonate or phosphorotihioate and triazole modification; and
   iii. modifications on the nucleic acid bases, such as on pyrimidines' C5-position and the N7 position of purines.
17. The nucleic acid aptamer of item 16, wherein at least one, preferably all, of the pyrimidine moieties of the nucleotide sequence are 2' substituted pyrimidines.
18. The nucleic acid aptamer of any of items 16 or 17, wherein the one or more 2' substitutions are selected from the group consisting of 2'-F (2'- Fluor), 2'-MOE (2'-Methoxyethyl) and 2'-OMe (2'-O-Methyl) .
19. A complex comprising the aptamer as defined in any one of the preceding items and one or more moieties.
20. The complex of item 19, wherein the aptamer is directly linked to the one or more moieties; or, alternatively, the aptamer is indirectly linked through a linker to one or more moieties.
21. The complex of any one of the items 19-20, wherein the moiety is selected from:
   (i) an antisense oligonucleotide (ASO), siRNA, microRNA, shRNA or a ribozyme;
   (ii) a moiety selected from a polysaccharide, a lipid, such as cholesterol or other lipid moieties, a non-proteinaceous polymer, such as one or more polyethylene glycol (PEG) chains, a polyamine, a peptide, a protein, a small molecule and combinations thereof;
   (iii) a label for detection purposes; preferably the label is selected from the group consisting of biotin, an enzyme, prosthetic group, fluorescent material, luminescent material, bioluminescent material, electron dense label, labels for magnetic resonance imaging, radioactive material, and combinations thereof;
   (iv) a drug;
   (vi) a nanoparticle; and
   (vii) combinations of any thereof.
22. The complex of any one of items 19-21, wherein the moiety is a siRNA, preferably a siRNA which recognizes a specific translocation product characterizing a EphA2 expressing cancer.
23. A composition comprising the aptamer of any one of items 1-18 or the complex of any one of items 19-22.
24. The composition of item 23, wherein said composition is a pharmaceutical composition comprising pharmaceutically acceptable excipients and/or carriers.
25. The aptamer of any one of items 1-18, the complex of any one of items 19-22 or the pharmaceutical composition of item 24, for use as a medicament.
26. Use of the aptamer of any one of items 1-18 or the complex of any one of items 19-22 as EphA2 detection or quantification agent; or as a cellular internalization vehicle targeting cells expressing EphA2.
27. The aptamer of any one of items 1-18, the complex of any one of the items 19-22 or the composition of any one of items 23 or 24, for use in a method of *in vivo* detection, such as an *in vivo* diagnostic method.
28. Use of the nucleic acid aptamer of any one of items 1-18, the complex of any one of items 19-22, or the composition of any one of items 23 or 24, as a detection agent in a method of *in vitro* or ex *vivo* detection, such as a diagnostic agent in a method of *in vitro* or ex *vivo* diagnosis.
29. A kit for the detection or quantification of EphA2 comprising the aptamer of any one of items 1-18, the complex of any one of items 19-22; or the composition of any one of items 23 or 24; and optionally means to detect the aptamer or complex.
30. A method for the detection or quantification of EphA2, wherein said method comprises a step of detecting or quantifying EphA2 using the aptamer according to any one of items 1-18, the complex of any one of items 19-22, the composition of any one of items 23 or 24, or the kit according to item 29.
31. A nucleic acid aptamer which binds specifically to EphA2, preferably human EphA2, the aptamer comprising the three polynucleotide regions Region 1, Region 2 and Region 3 defined below provided in the 5' to 3' order of the aptamer, wherein:
   i. Region 1 is of formula (R1):

      5' n₁ n₂ GA G n₆ n₇ C n₉ n₁₀ n₁₁ n₁₂ n₁₃ G G n₁₆ n₁₇ C 3'

      wherein:
      n₁ and n₂ are independently selected from G, dG or - ;
      n₆ is G, dG or -;
      n₇ is A, dA or -;
      n₉ is G, dG or -;
      n₁₀ is A, dA or -;
      n₁₁ is U, dU or -;
      n₁₂ is G, dG or -;
      n₁₃ is C, dC or -;
      n₁₆ is U, dU or -;
      n₁₇ is C, dC or -;
      wherein G, A, U and C are ribonucleotides; dG, dA, dU and dC are 2'-deoxyribonucleotides wherein unless otherwise indicated, the respective nucleotide includes the unmodified nucleotide and modified forms thereof, and "-" means absence of nucleotide;
      provided that
      n₆ and n₁₇ are complementary nucleotides or, alternatively, are absent;
      n₇ and n₁₆ are complementary nucleotides or, alternately, are absent;
      when n₁ is -, n₁₂ is -; and
      when n₇ is -, n₁₁ is U or dU;
   ii. Region 2 is of formula (R2):

      5' U G n₂₁ n₂₂ n₂₃ n₂₄ n₂₅ U G n₂₈ U C n₃₁ U C C C C A n₃₈ n₃₉ n₄₀ n₄₁ n₄₂ 3'

      wherein:
      n₂₁ is U, dU or -;
      n₂₂ is C, dC or -;
      n₂₃ is G, dG or -;
      n₂₄ is U, dU or -;
      n₂₅ is C, dC or -;
      n₂₈ is U, dU or -;
      n₃₁ is G, dG or -;
      n₃₈ is G, dG or-;
      n₃₉ is A, dA or -;
      n₄₀ is C, dC or -;
      n₄₁ is G, dG or -;
      n₄₂ is A, dA or -;
      provided that:
      n₂₁ and n₄₂ are complementary nucleotides or, alternatively, are absent;
      n₂₂ and n₄₁ are complementary nucleotides or, alternatively, are absent;
      n₂₃ and n₄₀ are complementary nucleotides or, alternatively, are absent;
      n₂₄ and n₃₉ are complementary nucleotides or, alternatively, are absent; and
      n₂₅ and n₃₈ are complementary nucleotides or, alternatively, are absent;
   iii. Region 3 is of formula (R3):

      5' C U C G C n₄₈ n₄₉ n₅₀ n₅₁ 3'

      wherein:
      n₄₈ is C, dC or -;
      n₄₉ is C, dC or -;
      n₅₀ is G, dG or-;
      n₅₁ is A, dA or-;
      provided that
      n₁ of R1 and n₄₉ of R3 are complementary nucleotides or, alternatively, are absent; and
      n₂ of R1 and n₄₈ of R3 are complementary nucleotides or, alternatively, are absent;
      or alternatively,
      a complex comprising the nucleic acid aptamer wherein the aptamer is directly linked to the one or more moieties; or, alternatively, the aptamer is indirectly linked through a linker to one or more moieties; or
      a pharmaceutical composition comprising the aptamer or the aptamer complex,
      for use in a method of *in vivo* detecting, such as *in vivo* diagnosing, or treating a EphA2-related disease in a subject.
32. Use of the nucleic acid aptamer, the complex or the composition as defined in item 31, as a diagnostic agent in a method of *in vitro* or ex *vivo* detection, such as *in vitro* or ex *vivo* diagnosis, of a EphA2-related disease.
33. The nucleic acid aptamer, the complex or the pharmaceutical composition for use according to item 31 or the use according to item 32, wherein said EphA2-related disease is a cancer characterized by expressing EphA2, preferably, wherein the cancer is selected from the group consisting of Ewing sarcoma, Ewing-like sarcoma, rhabdomyosarcoma, such as alveolar rhabdomyosarcoma, colon cancer, prostate cancer, breast cancer, ovary cancer, bladder cancer, esophageal carcinoma, pancreatic adenocarcinoma, brain cancer, head and neck cancer, cervical squamous cell carcinoma and endocervical adenocarcinoma.
34. The nucleic acid aptamer, the complex or the pharmaceutical composition for use in a method of treating a EphA2-related disease according to any one of items 31 or 33, wherein the aptamer, the complex or the pharmaceutical composition is administered in combination with another drug.
35. The nucleic acid aptamer, the complex or the pharmaceutical composition for use in a method of treating a EphA2-related disease according to item 34, wherein the aptamer, the complex or the pharmaceutical composition and the other drug are administered sequentially or simultaneously and are part of the same or separate compositions.
36. The nucleic acid aptamer, the complex or the pharmaceutical composition for use according to any one of items 31 and 33 to 36, or the use of items 32 or 33, wherein the nucleic acid aptamer, the complex and the composition are as defined item 31.
37. Use of a kit comprising the aptamer, the complex; or the composition as defined in item 31; and optionally means to detect the aptamer or complex; for the detection, such as the diagnosis, of a EphA2-related disease, preferably wherein said EphA2-related disease is a cancer characterized by expressing EphA2, such as, Ewing sarcoma, Ewing-like sarcoma, rhabdomyosarcoma, such as alveolar rhabdomyosarcoma, colon cancer, prostate cancer, breast cancer, ovary cancer, bladder cancer, esophageal carcinoma, pancreatic adenocarcinoma, brain cancer, head and neck cancer, cervical squamous cell carcinoma or endocervical adenocarcinoma.
38. A method for the detection, such as the diagnosis, of a EphA2-related disease cancer, wherein said method comprises a step of detecting or quantifying EphA2 using the aptamer, the complex, the composition or the kit according as defined in item 31; preferably wherein the EphA2-related disease is a cancer characterized by expressing EphA2, such as, Ewing sarcoma, Ewing-like sarcoma, rhabdomyosarcoma, such as alveolar rhabdomyosarcoma, colon cancer, prostate cancer, breast cancer, ovary cancer, bladder cancer, esophageal carcinoma, pancreatic adenocarcinoma, brain cancer, head and neck cancer, cervical squamous cell carcinoma or endocervical adenocarcinoma.

## Claims

1. A nucleic acid aptamer which binds specifically to EphA2, preferably human EphA2, the aptamer comprising the three polynucleotide regions Region 1, Region 2 and Region 3 defined below provided in the 5' to 3' order of the aptamer, wherein:
i. Region 1 is of formula (R1):
5' n₁ n₂ GA G n₆ n₇ C n₉ n₁₀ n₁₁ n₁₂ n₁₃ G G n₁₆ n₁₇ C 3'
wherein:
n₁ and n₂ are independently selected from G, or - ;
n₆ is G or
n₇ is A or
n₉ is G or
n₁₀ is A or -;
n₁₁ is U or-;
n₁₂ is G or-;
n₁₃ is C or-;
n₁₆ is U or-;
n₁₇ is C or-;
wherein G, A, U and C are ribonucleotides; including unmodified and modified forms thereof, and "-" means absence of nucleotide;
provided that
n₆ and n₁₇ are complementary nucleotides or, alternatively, are absent;
n₇ and n₁₆ are complementary nucleotides or, alternately, are absent;
when n₁ is -, n₁₂ is -; and
when n₇ is -, n₁₁ is U;
ii. Region 2 is of formula (R2):
5' U G n₂₁ n₂₂ n₂₃ n₂₄ n₂₅ U G n₂₈ U C n₃₁ U C C C C A n₃₈ n₃₉ n₄₀ n₄₁ n₄₂ 3'
wherein:
n₂₁ is U or-;
n₂₂ is C or-;
n₂₃ is G or-;
n₂₄ is U or-;
n₂₅ is C or-;
n₂₈ is U or-;
n₃₁ is G or-;
n₃₈ is G or-;
n₃₉ is A or -;
n₄₀ is C or -;
n₄₁ is G or -;
n₄₂ is A or -;
provided that:
n₂₁ and n₄₂ are complementary nucleotides or, alternatively, are absent;
n₂₂ and n₄₁ are complementary nucleotides or, alternatively, are absent;
n₂₃ and n₄₀ are complementary nucleotides or, alternatively, are absent;
n₂₄ and n₃₉ are complementary nucleotides or, alternatively, are absent; and
n₂₅ and n₃₃ are complementary nucleotides or, alternatively, are absent;
iii. Region 3 is of formula (R3):
5' C U C G C n₄₈ n₄₉ n₅₀ n₅₁ 3'
wherein:
n₄₈ is C or -;
n₄₉ is C or -;
n₅₀ is G or-;
n₅₁ is A or -;
provided that
n₁ of R1 and n₄₉ of R3 are complementary nucleotides or, alternatively, are absent; and
n₂ of R1 and n₄₈ of R3 are complementary nucleotides or, alternatively, are absent;
wherein said aptamer is further **characterized by**:
a) having a length of 50 nucleotides or less; and/or
b) n₁₁ and/or n₁₂ of R1 is absent.

2. The nucleic acid aptamer of claim 1, which comprises or consists of the sequence of formula (I):
5' n₁ n₂ G A G n₆ n₇ C n₉ n₁₀ n₁₁ n₁₂ n₁₃ G G n₁₆ n₁₇ C U G n₂₁ n₂₂ n₂₃ n₂₄ n₂₅ U G n₂₈ U C n₃₁ U C C C C A n₃₈ n₃₉ n₄₀ n₄₁ n₄₂ C U C G C n₄₈ n₄₉ n₅₀ n₅₁, 3,
wherein nucleotides n₁, n₂, n₆, n₇, n₉ to n₁₃, n₁₆, n₁₇, n₂₁ to n₂₅, n₂₈, n₃₁, n₃₈ to n₄₂ and n₄₈ to n₅₁ are as defined in claim 1, preferably wherein n₂₅ is C and n₂₈ is U.

3. The nucleic acid aptamer of claim 1 or 2, wherein the aptamer is selected from the group consisting of the sequences identified in Table 2.1 and/or Table 2.2, or a variant having at least 85%, preferably at least 90% identity, more preferably at least 95% identity to any thereof.

4. A complex comprising the aptamer as defined in any one of the preceding claims and one or more moieties, preferably the moiety is selected from:
(i) an antisense oligonucleotide (ASO), siRNA, microRNA, shRNA or a ribozyme;
(ii) a moiety selected from a polysaccharide, a lipid, such as cholesterol or other lipid moieties, a non-proteinaceous polymer, such as one or more polyethylene glycol (PEG) chains, a polyamine, a peptide, a protein, a small molecule and combinations thereof;
(iii) a label for detection purposes; preferably the label is selected from the group consisting of biotin, an enzyme, prosthetic group, fluorescent material, luminescent material, bioluminescent material, electron dense label, labels for magnetic resonance imaging, radioactive material, and combinations thereof;
(iv) a drug;
(vi) a nanoparticle; and
(vii) combinations of any thereof.

5. A composition comprising the aptamer of any one of claims 1-3 or the complex of claim 4, preferably wherein said composition is a pharmaceutical composition comprising pharmaceutically acceptable excipients and/or carriers.

6. The aptamer of any one of claims 1-3, the complex of claim 4 or the composition of claim 5, for use as a medicament, alone or as a combination therapy.

7. The aptamer of any one of claims 1-3, the complex of claim 4 or the composition of claim 5, for use in a method of *in vivo* detection or quantification of a target protein or for use in an *in vivo* method for the detection or prognosis of a disease.

8. Use of the nucleic acid aptamer of any one of claims 1-3, the complex of claim 4, or the composition of claim 5, as a detection or quantification agent in a method of *in vitro* or ex *vivo* detection or quantification of a target protein or in an *in vitro* or ex *vivo* method for the detection or prognosis of a disease.

9. A kit for the detection or quantification of EphA2 comprising the aptamer of any one of claims 1-3, the complex of claim 4; or the composition of claim 5; and optionally means to detect the aptamer or complex.

10. A nucleic acid aptamer which binds specifically to EphA2, preferably human EphA2, the aptamer comprising the three polynucleotide regions Region 1, Region 2 and Region 3 defined below provided in the 5' to 3' order of the aptamer, wherein:
i. Region 1 is of formula (R1):
5' n₁ n₂ GA G n₆ n₇ C n₉ n₁₀ n₁₁ n₁₂ n₁₃ G G n₁₆ n₁₇ C 3'
wherein:
n₁ and n₂ are independently selected from G, or - ;
n₆ is G or-;
n₇ is A or -;
n₉ is G or-;
n₁₀ is A or -;
n₁₁ is U or -;
n₁₂ is G or -;
n₁₃ is C or-;
n₁₆ is U or -;
n₁₇ is C or-;
wherein G, A, U and C are ribonucleotides, including unmodified and modified forms thereof, and "-" means absence of nucleotide;
provided that
n₉ and n₁₇ are complementary nucleotides or, alternatively, are absent;
n₇ and n₁₆ are complementary nucleotides or, alternately, are absent;
when n₁ is -, n₁₂ is -; and
when n₇ is -, n₁₁ is U;
ii. Region 2 is of formula (R2):
5' U G n₂₁ n₂₂ n₂₃ n₂₄ n₂₅ U G n₂₈ U C n₃₁ U C C C C A n₃₈ n₃₉ n₄₀ n₄₁ n₄₂ 3'
wherein:
n₂₁ is U or -;
n₂₂ is C or -;
n₂₃ is G or -;
n₂₄ is U or -;
n₂₅ is C or -;
n₂₈ is U or -;
n₃₁ is G or-;
n₃₈ is G or-;
n₃₉ is A or -;
n₄₀ is C or -;
n₄₁ is G or -;
n₄₂ is A or -;
provided that:
n₂₁ and n₄₂ are complementary nucleotides or, alternatively, are absent;
n₂₂ and n₄₁ are complementary nucleotides or, alternatively, are absent;
n₂₃ and n₄₀ are complementary nucleotides or, alternatively, are absent;
n₂₄ and n₃₉ are complementary nucleotides or, alternatively, are absent; and
n₂₅ and n₃₈ are complementary nucleotides or, alternatively, are absent;
iii. Region 3 is of formula (R3):
5' C U C G C n₄₈ n₄₉ n₅₀ n₅₁ 3'
wherein:
n₄₈ is C or -;
n₄₉ is C or -;
n₅₀ is G or-;
n₅₁ is A or -;
provided that
n₁ of R1 and n₄₉ of R3 are complementary nucleotides or, alternatively, are absent; and
n₂ of R1 and n₄₈ of R3 are complementary nucleotides or, alternatively, are absent;
or alternatively,
a complex comprising the nucleic acid aptamer wherein the aptamer is directly linked to the one or more moieties; or, alternatively, the aptamer is indirectly linked through a linker to one or more moieties; or
a composition comprising the aptamer or the aptamer complex,
for use in
a method of *in vivo* detecting or quantifying EphA2; or
a method comprising *in vivo* detection or quantification of EphA2 or EphA2-expressing cells for the screening, diagnosis, prognosis, or monitoring of a EphA2- related disease , or
a method for treating cancer or a EphA2-related disease in a subject;
preferably, wherein said EphA2-related disease is a cancer **characterized by** expressing EphA2, preferably, wherein the cancer is selected from the group consisting of Ewing sarcoma, Ewing-like sarcoma, rhabdomyosarcoma, such as alveolar rhabdomyosarcoma, colon cancer, prostate cancer, breast cancer, ovary cancer, bladder cancer, esophageal carcinoma, pancreatic adenocarcinoma, brain cancer, head and neck cancer, cervical squamous cell carcinoma and endocervical adenocarcinoma.

11. Use of the nucleic acid aptamer or the complex as defined in claim 10, as EphA2 detection or quantification agent; or as a cellular internalization vehicle targeting cells expressing EphA2.

12. Use of the nucleic acid aptamer, the complex or the composition as defined in claim 10, as a detection or quantification agent in a method of
*in vitro or ex vivo* detection or quantification of EphA2, or
in a method comprising the *in vitro or ex vivo* detection or quantification of EphA2 for the screening, diagnosis, prognosis, or monitoring of a EphA2-related disease;
preferably, wherein said EphA2-related disease is a cancer **characterized by** expressing EphA2, preferably, wherein the cancer is selected from the group consisting of Ewing sarcoma, Ewing-like sarcoma, rhabdomyosarcoma, such as alveolar rhabdomyosarcoma, colon cancer, prostate cancer, breast cancer, ovary cancer, bladder cancer, esophageal carcinoma, pancreatic adenocarcinoma, brain cancer, head and neck cancer, cervical squamous cell carcinoma and endocervical adenocarcinoma.

13. The nucleic acid aptamer, the complex or the pharmaceutical composition for use in a method of treating a EphA2-related disease according to claim 10 or a use according to claim 12, wherein the aptamer, the complex or the pharmaceutical composition is administered in combination with another drug.

14. Use of a kit comprising the aptamer, the complex; or the composition as defined in claim 10; and optionally means to detect the aptamer or complex; for the detection or quantification of EphA2,
in a method for the *in vitro or ex vivo* detection or quantification of EphA2, or in a method comprising the *in vitro or ex vivo* detection or quantification of EphA2 for the screening, diagnosis, prognosis, or monitoring of a EphA2-related disease,
preferably wherein said EphA2-related disease is a cancer **characterized by** expressing EphA2, such as, Ewing sarcoma, Ewing-like sarcoma, rhabdomyosarcoma, such as alveolar rhabdomyosarcoma, colon cancer, prostate cancer, breast cancer, ovary cancer, bladder cancer, esophageal carcinoma, pancreatic adenocarcinoma, brain cancer, head and neck cancer, cervical squamous cell carcinoma or endocervical adenocarcinoma.

15. A process for producing a nucleic acid aptamer of any one of claims 1 to 3, wherein said process comprises synthesizing the aptamer by standard solid phase synthesis using the phosphoramidite method; by *in vitro* transcription using standard or modified retrotranscriptases or as a direct result of the Systematic Evolution of Ligands by Exponential Enrichment (SELEX) process.

16. A process for producing a complex of claim 4, wherein said process comprises:
(i) providing a nucleic acid aptamer of the first aspect of the invention; and
(ii) directly or indirectly linking the aptamer to one or more moieties.
